# EUROPEAN PATENT SPECIFICATION

(11) **EP 3 995 494 B1**
(45) Date of publication and mention of the grant of the patent: **18.06.2025**
(21) Application number: 20206285.7
(22) Date of filing: 06.11.2020
(51) Int. Cl.: C07D 405/12, C07D 405/14, C07D 231/12, A61K 31/415, A61P 35/00

(54) **PROTEIN-PROTEIN INTERACTION MODULATORS OF AURORA KINASE A AND THEIR USE IN THE PREVENTION AND/OR TREATMENT OF CANCER**
MODULATOREN DER PROTEIN-PROTEIN-INTERAKTION DER AURORA-KINASE A UND DEREN VERWENDUNG IN DER VORBEUGUNG UND/ODER BEHANDLUNG VON KREBS
MODULATEURS D'INTERACTIONS PROTÉINE-PROTÉINE DE L'AURORA KINASE A ET LEUR UTILISATION DANS LA PRÉVENTION ET/OU LE TRAITEMENT DU CANCER

(43) Date of publication of application: 11.05.2022
(73) Proprietor: Eberhard Karls Universität Tübingen, 72074 Tübingen (DE)
(72) Inventor: Reiner, Juliander J., 72070 Tübingen (DE); Pantsar, Tatu, 70771 Leinfelden-Echterdingen (DE); Flötgen, Dirk, 53129 Bonn (DE); Laufer, Stefan, 72070 Tübingen (DE); Zender, Lars, 72108 Rottenburg (DE); Henning, Melanie, 72070 Tübingen (DE)
(74) Representative: Kuttenkeuler, David

(56) References cited:
- EP-A1- 2 754 659
- WO-A1-03/013517
- WO-A2-2005/113494
- MOHANE SELVARAJ COUMAR ET AL: "Fast-Forwarding Hit to Lead: Aurora and Epidermal Growth Factor Receptor Kinase Inhibitor Lead Identification", JOURNAL OF MEDICINAL CHEMISTRY, vol. 53, no. 13, 8 July 2010 (2010-07-08), pages 4980 - 4988, XP055767500, ISSN: 0022-2623, DOI: 10.1021/jm1000198
- CHEUNG CHUN HEI ANTONIO ET AL: "BPR1K653, a Novel Aurora Kinase Inhibitor, Exhibits Potent Anti-Proliferative Activity in MDR1 (P-gp170)-Mediated Multidrug-Resistant Cancer Cells", PLOS ONE, vol. 6, no. 8, 24 January 2011 (2011-01-24), pages e23485, XP055788186, DOI: 10.1371/journal.pone.0023485
- CHANG HSU YUNG ET AL: "Discovery of BPR1K871, a quinazoline based, multi-kinase inhibitor for the treatment of AML and solid tumors: Rational design, synthesis, in vitro and in vivo evaluation", ONCOTARGET, vol. 7, no. 52, 27 December 2016 (2016-12-27), United States, pages 86239 - 86256, XP055788199, ISSN: 1949-2553, DOI: 10.18632/oncotarget.13369

## Description

### FIELD OF THE INVENTION

The present invention relates to compounds that modulate the conformation of Aurora Kinase A (AURKA). The compounds of the present invention are also modulators of the interactome of AURKA, and preferably alter the protein-protein interaction of Aurora Kinase A (AURKA) with binding proteins, such as MYC and/or TPX2. The present invention also pertains to the use of such compounds in the prevention and/or treatment of proliferative diseases, such as cancer, and kits comprising the same.

### DESCRIPTION

Cancer is a major lethal disease for humans and is caused by physiologically-uncontrolled proliferation of cancer cells. Despite tremendous efforts on cancer studies and treatments over the past decades, cancer remains one of the most common causes of death in the world. Cancer affects a multitude of normal physiological conditions of the human body, resulting in serious pathological reactions that often lead to the death of the patient.

Primary liver cancer represents the sixth most common cancer incident and the fourth most common cause for cancer related deaths worldwide. Currently, the best way to treat liver cancer is by means of surgical resection and liver transplantation. However, in the case of liver transplantation, it is difficult to find donors, whereas surgical resection is not an option for all patients, but is limited to around 20% of all patients. Other treatment methods include systemic chemotherapy, hepatic artery chemoembolization, hepatic artery chemotherapy infusion, and radiotherapy, but these treatments not only kill liver cancer cells but also normal cells, giving rise to fatal side effects.

Hepatocellular carcinoma (HCC) constitutes the majority of primary liver cancers, but the only therapeutic option for late stage HCC is a systemic treatment with kinase inhibitors, such as sorafenib, or the PD-L1 inhibitor Nivolumab. However, such treatment only provides a very limited prolonged survival of patients with late stage HCC due to the fast recurrence of therapy resistant tumors. For example, sorafenib merely provides on average 2.8 months prolonged survival. Moreover, treatment with sorafenib has caused severe side effects, such as a reduced pancreatic function and size. Therefore, novel therapeutic approaches for liver cancer, in particular late stage HCC, are urgently needed.

Lung cancer is the most common cancer worldwide, and among all cancer types the most common cause of death (resulting in 20.4% of all cancer deaths). About 318,000 new cases are diagnosed every year in the EU alone, with 85% of the patients presenting with advanced disease resulting in an overall 5-year survival of only 5% to 15%. The major risk factor for lung cancer is cigarette smoking, contributing to 80% of the overall attributable risk. Other risks are exposure to asbestos and radon and, to some extent, genetic predisposition.

Lung cancer can be subdivided into four major histological types: small cell lung carcinoma (SCLC), adenocarcinoma, squamous cell carcinoma and large cell carcinoma. The term non-small cell lung carcinoma (NSCLC) refers to the latter three types of lung cancer, i.e. adenocarcinoma, squamous cell carcinoma and large cell carcinoma, and NSCLC accounts for 80% of all lung cancers. Adenocarcinoma arise from alveolar or bronchiolar epithelial or mucin-producing cells, while squamous cell carcinoma is derived from bronchial epithelium. Large cell carcinoma is a less differentiated form without an apparent glandular of squamous cell phenotype.

SCLC accounts for the remaining 20% of lung cancers, originating from epithelial cells with neuro-endocrine features. Alterations in the tumor suppresser gene *TP53,* coding for p53, are the most frequent alterations found in lung cancer. Mutations in *TP53* are found in about 90% of SCLC and about 50% of NSCLC tumors. Additionally, certain mutational hotspots correlate with exposure to tobacco smoke. Concurrent alterations in both of the tumor suppresser genes *TP₅₃* and RB transcriptional corepressor 1 (*RB1*) are also often found in lung cancer, in particular in SCLC. SCLC progresses rapidly and is usually only detected in greatly advanced stages.

Current therapeutic options for most cancer diseases, including lung cancer, in particular SCLC, include radiation therapy to destroy cancer cells, chemotherapy to prevent tumorous cells from growing and dividing further, immunotherapy to boost the body's natural defense system to fight the cancer, and surgery to remove the tumorous tissue during an operation. However, all of these treatment strategies have severe side effects. Moreover, many cancers become resistant to treatment, or patients relapse following treatment. Furthermore, in patients treated with a chemotherapeutic agent, a continuous selection pressure exists to develop cancer cell clones which are resistant to the chemotherapeutic used.

Aurora kinase A (AURKA) is a serine/threonine-protein kinase that plays a key role during mitosis and meiosis. AURKA's enzymatic activity is highest during the G2 phase to M phase transition in the cell cycle, and its function is essential for a normal cell proliferation. AURKA is required for the recruitment of several important proteins necessary for the formation of the mitotic spindle, and AURKA is also necessary for the proper separation of the centrosomes after the mitotic spindle has been formed. Interestingly, AURKA has been shown to be dysregulated in several different cancer diseases, and AURKA is known to interact with multiple proteins, including MYC, P53, and TPX2.

TPX2, also known as targeting protein for Xklp2, is a microtubule assembly factor that plays a key role in microtubule nucleation and growth during the mitotic phase. TPX2 is also important in activating and recruiting AURKA during mitosis. In the presence of nuclear import factor importin α, TPX2 is unable to bind to AURKA, though it is still able to bind microtubules via its amino-terminal domain, which leads to inhibition of M phase microtubule nucleation. TPX2 recruits and activates AURKA by utilizing its short 43 amino acid long amino-terminal sequence to bind the catalytic domain of AURKA, locking the kinase into its active conformation. Importantly, the interaction between AURKA and TPX2 is essential for the formation of the mitotic spindle. TPX2 is known to be overexpressed in different types of cancer including, but not limited thereto, hepatocellular carcinoma (HCC), medullary thyroid cancer, bladder carcinoma, and metastatic breast cancer.

MYC (also known as c-MYC) is a regulator gene and proto-oncogene that codes for a transcription factor, which regulates the expression of a multitude of genes. MYC is often constitutively expressed in cancer, leading to the increased expression of many genes, some of which are involved in cell proliferation, which is instrumental for the formation of cancer. MYC is dysregulated in 30-60 % of HCCs, and transgenic mouse models have demonstrated that MYC amplification is a driving event in HCC genesis, usually accompanied by an addiction to MYC overexpression. Soucek *et al.* genetically modeled the transient systemic inhibition of MYC in a preclinical mouse model via conditional expression of a dominant-negative MYC mutant (OmoMYC). Metronomic cycles of OmoMYC expression resulted in long-term tumor control, while only moderate and fully reversible adverse effects on normal proliferating tissues were observed. Importantly, reoccurring tumors remained sensitive to MYC inhibition.

Direct targeting of MYC by small molecules, however, is challenging as no conventional binding cavities are present. The mainly intrinsically disordered MYC protein contains only one ordered domain, a basic helix-loop-helix (bHLH) with a leucine-zipper that enables DNA binding upon dimerization with other transcription factors, such as MYC-associated factor X (MAX). Unfortunately, direct inhibition of MYC or the MYC-MAX heterodimer have not been successful.

Accordingly, there is an urgent need for indirect methods for targeting MYC. Interestingly, one promising approach for indirect MYC targeting is based on an AURKA mediated protection mechanism, which was initially shown for N-MYC. AURKA and the FbxW7 subunit of the SCF^{FbxW7} ubiquitin ligase complex compete for N-MYC binding, whereby its ubiquitination and subsequent proteasomal degradation is prevented. Importantly, the dependency on a direct interaction between AURKA and N-MYC was demonstrated, whereas inhibition of AURKAs catalytic activity was not required.

Due to the dysregulation of AURKA in many types of cancer, AURKA inhibitors have been developed and extensively studied, including the AURKA inhibitors alisertib and CD532. Interestingly, alisertib and CD532 have been shown to not only inhibit the enzymatic activity of AURKA, but also induce a conformational shift in AURKA. This conformational shift has then been disclosed to block the protein-protein interaction between both N-MYC and MYC with AURKA, which prevents N-MYC / MYC stabilization by AURKA, consequently leading to N-MYC / MYC degradation *in vitro* and *in vivo.*

Interestingly, using a mouse model it was shown that the development of Nras-driven, and *Trp53*-deficient HCC, depends on MYC and its stabilization by AURKA, representing a promising strategy for indirect MYC targeting via targeting the AURKA-MYC complex protective mechanism. Accordingly, *Nras*-driven, *Trp53*-deficient murine HCCs are sensitive to the AURKA inhibitors alisertib and CD532, which induce a conformational shift in AURKA, whereas conventional AURKA kinase inhibitors that do not induce a conformational shift in AURKA showed no effect in these cells. Treatment with CD532 and alisertib resulted in tumor cell death *in vitro* and *in vivo,* while *Nras*-driven, *Cdkn2a^{ARF}*-deficient murine HCCs, which develop independently of MYC, showed no response.

WO2005113494 (A2) relates to compounds that are capable of modulating kinase enzymes thereby influencing the process of angiogenesis and treating angiogenesis-related diseases and other proliferative disorders, including cancer and inflammation. Additionally, pharmaceutical compositions are provided, including the compounds, and methods of treating diseases related to the activity of protein kinases.

Mohane et al disclose the identification of several synthetically tractable and structurally distinct leads for Aurora and EGFR kinase inhibition through rapid construction of a furanopyr-imidine kinase-targeted library and screening it without isolation of the compounds. Furthermore, potent bits were identified from the library and further modified based on structure biology/docking studies in the kinase.

Cheung et al disclose BPR1K653 as a potent pan-Aurora kinase inhibitor that is able to target cancer cells regardless of their tissue origins, MDRI or p53 Status. These key features are supposed to distinguish this compound from other previously developed Aurora kinase inhibitors and anti-cancer compounds. At the molecular level, BPR1K653 can be used as a tool to study the molecular functions of Aurora kinases in the MDR1-induced drug resistant cancer cells in the future.

Yung et al disclose the design and synthesis of a quinazoline-based, multi-kinase inhibitor for the treatment of acute myeloid leukemia (AML} and other malignancies. Kinase profiling and cell-line profiling revealed BPR1K871 to be a potential multi-kinase inhibitor.EP 2 754 659 (A1) provides derivatives substituted by urea associated with 4-substituted-(S-substituted-1H-pyrazole-5-amino)-pyrimidine-2-amino of formula (I), wherein these compounds may selectively regulate or inhibit an information transmission process controlled by natural or variant tyrosine kinase. Also provided are preparation methods and uses of the compounds.

WO 03/013517 (A1) relates to compounds, which are aminoisoxazole derivatives or pharmaceutically acceptable salts thereof, together with pharmaceutical compositions comprising them are disclosed; these compounds or compositions are useful in the treatment of diseases caused by and/or associated with an altered protein kinase activity such as cancer, cell proliferative disorders, Alzheimer's disease, viral infections, auto-immune diseases and neurodegenerative disorders.

WO2008063525 (A1) provides inhibitors of AURKA, pharmaceutical compositions comprising the compounds, and methods of using the compounds for the treatment of cancer.

WO2011103089 (A1) relates to sodium 4-{[9-chloro-7-(2-fluoro-6-methoxyphenyl)-5H-pyrimido[5,4-d][2]benzazepin-2-yl]amino}-2-methoxybenzoate, as well as its use as an Aurora kinase inhibitor, pharmaceutical compositions comprising the crystalline forms of the compound, and methods of making such pharmaceutical compositions.

WO2014190207 (A1) discloses AURKA inhibitors that disrupt the native conformation of AURKA and cause degradation of N-MYC in N-MYC-expressing neuroblastoma cell lines, such as the compound CD532.

However, CD532 has later been shown to induce proteasomal degradation of AURKA in vivo, undermining a selective therapeutic effect. Moreover, CD532 is significantly competing with ATP for binding to AURKA, thereby leading to a strong inhibition of the kinase activity of AURKA. Since the majority of AURKAs' functions regarding normal mitosis are dependent on its catalytic activity, the inhibition of the kinase activity of AURKA is causative for severe side effects. Accordingly, AURKA inhibitors were found to display dose limiting toxicities, including anemia and neutropenia, and clinical studies of, for example, alisertib (MLN8237) for patients with relapsed or refractory peripheral T-cell lymphoma (PTCL) and patients with small cell lung cancer (SCLC) were then terminated.

These drawbacks of existing AURKA inhibitors reveal a high demand for developing next generation compounds targeting the AURKA interactome, wherein said compounds are characterized by causing fewer side effects upon administration in a subject, such as a patient. In view of the above, new and effective treatments for cancer, such as hepatocellular carcinoma (HCC) and/or small cell lung cancer (SCLC), as well as novel strategies to fight cancer, are also urgently needed.

Thus, it is an object of the present invention to provide compounds that modulate the conformation of AURKA. It is a further object of the invention to provide modulators of the protein-protein interaction of AURKA with binding proteins, such as MYC and/or TPX2. A further problem to be solved by the present invention is to provide modulators of the protein-protein interaction of AURKA with binding proteins, such as MYC and/or TPX2, that show a weakened or absent inhibition of the kinase activity of AURKA. Yet another problem to be solved by this invention is to provide compounds, which are useful for treating cancer diseases, such as HCC and SCLC. Hence, the present invention is based on novel compounds that solve the above problems, and pharmaceutical compositions comprising the same.

### BRIEF DESCRIPTION OF THE INVENTION

Generally, and by way of brief description, the main aspects of the present invention can be described as follows:

In a first aspect, the invention pertains to a compound comprising an anilide moiety according to formula (III).

In a second aspect, the invention pertains to a pharmaceutical composition comprising a compound comprising an anilide moiety according to formula (III).

In a third aspect, the invention pertains to a compound comprising an anilide moiety according to formula (III), or a pharmaceutical composition comprising the same, for use in medicine.

In a fourth aspect, the invention pertains a compound comprising an anilide moiety according to formula (III), or a pharmaceutical composition comprising the same, for use in the prevention and/or treatment of a proliferative disease, such as cancer.

In a fifth aspect, the invention pertains to a kit comprising a compound according to this invention, or a pharmaceutical composition comprising the same.

In a sixth aspect, the invention pertains to the in vitro use of a compound comprising an anilide moiety according to formula (III), a pharmaceutical composition or a kit comprising the same, in selectively modulating the interaction of a serine/threonine kinase, or a variant thereof, with at least one binding protein.

In a seventh aspect, the invention pertains to the in vitro use of a compound comprising an anilide moiety according to formula (III), a pharmaceutical composition or a kit comprising the same, in modulating the AURKA interactome.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The above problem is solved in a first aspect by providing a compound, wherein the compound comprises an anilide moiety according to formula (III): 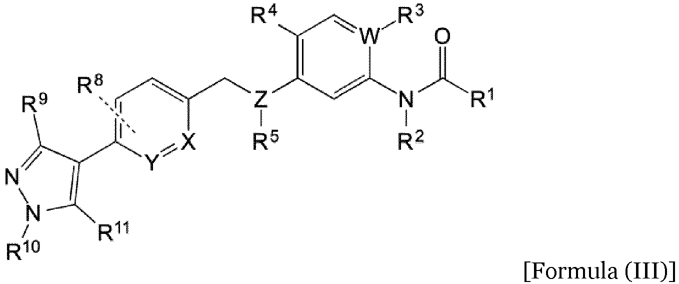 wherein
- W: is C, or N, if W = N, R³ is dispensed with;
- X and Y: are independently selected from C and N;
- Z: is N, or O; if Z = O, R⁵ is dispensed with;
- R¹: is selected from -CH₂-aryl; -CH₂-(hetero)aryl; -CH₂-CH₂-aryl; -CH₂-CH₂-(hetero)aryl; -N(R²)-CH2-aryl; and -N(R²)-CH2-heteroaryl
- R²: is H, or Me;
- R³ and R⁴: are independently selected from H, F, Cl and Me;
- R⁵: is H;
- R⁸: is one or more substituents selected from H, F and methoxy, preferably two F or two methoxy substituents;
- R⁹: is H, or alkyl;
- R¹⁰: is selected from alkyl, aminoalkyl, morpholinalkyl, acetyl, tosyl, and benzyl;
- R¹¹: is H, NH₂, or aminobenzyl;
and the pharmaceutically acceptable salts, solvates and optical isomers thereof.

The term "anilide" as used herein, shall refer to an amide of aniline. It was surprisingly found that a compound comprising an anilide moiety according to formula (III) solves the above problems by specifically modulating the conformation of AURKA, thereby modifying the protein-protein interaction of AURKA with binding proteins, such as MYC and/or TPX2. Simultaneously, the use of a compound comprising an anilide moiety according to formula (III) as above is characterized by a largely reduced or no inhibition of the kinase activity of AURKA, compared to previously studied AURKA inhibitors, making the novel compounds comprising an anilide moiety according to formula (III) of this invention useful for treating various cancer diseases, such as HCC and SCLC.

In a preferred embodiment, R² and R⁴ of the compound according to this invention, in particular a compound comprising an anilide moiety according to formula (III), and the pharmaceutically acceptable salts, solvates and optical isomers thereof, is H.

In still a further embodiment, R³ of the compound according to this invention, in particular a compound comprising an anilide moiety according to formula (III), and the pharmaceutically acceptable salts, solvates and optical isomers thereof, is F.

The term "compound" as used herein, shall not be understood as being limited to the compound itself, but shall also refer to any pharmaceutically acceptable salt, solvate and/or optical isomer of the particular compound. Rather than being limited to the compound itself, the term "compound" also comprises pharmaceutically acceptable salts, solvates and optical isomers of the compound, and even pharmaceutical compositions comprising the compound.

In a particularly preferred embodiment, R¹ of the compound according to this invention, in particular a compound having formula (III), and the pharmaceutically acceptable salts, solvates and optical isomers thereof, is selected from phenethyl, methyl, and (furan-2-yl)ethyl. Particularly preferred is that R¹ is methyl or (furan-2-yl)ethyl.

Yet another further preferred embodiment relates to a compound according to this invention, in particular a compound having formula (III), and the pharmaceutically acceptable salts, solvates and optical isomers thereof, wherein Z is N and R⁵ is H.

In a further embodiment, X and Y of the compound according to this invention, in particular a compound having formula (III), and the pharmaceutically acceptable salts, solvates and optical isomers thereof, are C. A further embodiment relates to a compound according to this invention, in particular a compound having formula (III), and the pharmaceutically acceptable salts, solvates and optical isomers thereof, wherein X is C and Y is N. Yet another embodiment relates to a compound according to this invention, in particular a compound having formula (III), and the pharmaceutically acceptable salts, solvates and optical isomers thereof, wherein X is N and Y is C.

Yet another further preferred embodiment relates to a compound according to this invention, in particular a compound having formula (III), and the pharmaceutically acceptable salts, solvates and optical isomers thereof, wherein R⁸ merely contains H as substituents.

In still a further embodiment, R⁹ and R¹¹ of the compound according to this invention, in particular a compound having formula (III), and the pharmaceutically acceptable salts, solvates and optical isomers thereof, are H.

In a further embodiment, R¹⁰ of the compound according to this invention, in particular a compound having formula (III), and the pharmaceutically acceptable salts, solvates and optical isomers thereof, is H or methyl.

A particularly useful and preferred compound according to this invention is [*N*-(5-((4-(1*H*-pyrazole-4-yl)benzyl)amino)-2-fluorophenyl)-3-(furan-2-yl)propanamide], or the pharmaceutically acceptable salts, solvates and optical isomers thereof.

Yet another particularly preferred embodiment relates to a compound according to this invention, which is [*N*-(2-fluoro-5-((4-(1-methyl-1*H*-pyrazole-4-yl)benzyl)amino)phenyl)-3-(furan-2-yl)propanamide], or the pharmaceutically acceptable salts, solvates and optical isomers thereof.

Further, the invention relates to compounds comprising an anilide moiety according to formula (III), in which the variables are as defined above.

Additionally, the invention shall further include any pharmaceutically acceptable salt of the compounds mentioned above. The pharmaceutically acceptable salts are either acid or base addition salts of a compound as above, with pharmaceutically acceptable acids or bases. According to this invention, any suitable pharmaceutically acceptable organic and inorganic acid may be used, such as, for example, sulfuric acid, sulfamic acid, hydrochloric acid, hydrobromic acid, phosphoric acid, dicarboxylic acids, tricarboxylic acids and hydroxycarboxylic acids having 2 to 10 carbon atoms, such as oxalic acid, malonic acid, maleic acid, fumaric acid, lactic acid, citric acid, and benzoic acid, CrC₄-alkylsulfonic acids, such as methanesulfonic acid, cycloaliphatic sulfonic acids, such as S-(+)-10-camphor sulfonic acid, or aromatic sulfonic acids, such as benzenesulfonic acid and toluenesulfonic acid. Further, any suitable pharmaceutically acceptable organic and inorganic base may be used according to this invention, such as, for example, ammonium hydroxide, alkali metal hydroxides, such as sodium hydroxide or potassium hydroxide, alkaline earth metal hydroxides, such as calcium or magnesium hydroxide, or organic nitrogen bases, such as dimethylamine, trimethylamine, ethanolamine, diethanolamine, triethanolamine, choline, procaine, or ethylenediamine.

According to the present invention, solvates of the above compounds are also within the scope of this invention. Examples of solvates are hydrates, but any other solvate shall also be included.

It is to be understood that in any embodiment of the invention, where the compound comprising an anilide moiety according to formula (III), comprises at least one chiral center, e.g. an asymmetric carbon atom, the invention extends to all the optical isomers thereof, as well as to racemates and to isomer mixtures thereof, wherein any isomer or any number of isomers may be present in any amount. Examples of optical isomers are enantiomers and diastereomers, which shall also be included in the present invention. Further, the invention also extends to all tautomeric, crystal and/or polymorphic forms of the compounds comprising an anilide moiety according to formula (III), or any other compound of this invention.

According to the present invention, prodrugs of the compounds above shall also be included. The term "prodrug" or "pro-drug" shall relate to a compound or an agent that can be transformed into the actual active compound, preferably in the present context into any of the compounds of the invention. Such transformation usually occurs in the human or animal body through a physicochemical transformation process, such as an enzymatic or chemical cleavage of a moiety. Candidate enzymes that could be utilized to activate the prodrugs according to the present invention include lipases, proteases or glycosidases. Importantly, prodrugs can be designed so that the transformation process occurs in particular tissues or regions of the human or animal body. Without being limited thereto, an example of a prodrug of a compound according to the present invention is any compound of the invention as above in form of an ester, or a prodrug comprising a carboxylic acid substituent wherein the free hydrogen is replaced by an alkyl, such as alkoxycarbonyloxymethyl having from 3 to 6 carbon atoms, 1-methyl-1-(alkoxycarbonyloxy)-ethyl having from 5 to 8 carbon atoms, (Cᵢ-C₆)alkanoyloxy-methyl, piperidino-, pyrrolidino- or morpholino(C₂-C₃)alkyl, or arylactyl.

According to the present invention, it might be beneficial to use a prodrug of a compound of this invention, because said prodrug might be more soluble in a liquid, such as more water soluble, than the ultimate active compound. Also, a different route of administration available for a pro-drug might be beneficial over the required route of administration of the final drug. For example, a pro-drug may be administrable by intravenous administration. Moreover, the penetration of certain organs might differ between final drug and prodrug. For example, a prodrug might be able to cross the blood-brain-barrier, whereas the active end-drug might not. Also, bioavailability of a prodrug might be advantageous over bioavailability of the final drug.

According to the present invention, the prefix Cₙ-Cₘ indicates in each case the possible number of carbon atoms in the group. As used herein, the term "halogen" shall be used as collective term and shall include any halogen, such as fluorine, chlorine, bromine, or iodine. Preferably, the term halogen shall refer to fluorine or chlorine. Similarly to the term halogen, any organic moiety of a variable of a compound according to the present invention as defined above shall be understood as collective term for individual listings of the individual group members.

The term "carbonyl" shall refer to a group having at least 1, preferably more, carbon atoms.

The term "aminocarbonyl", as used herein, shall refer to the group NH₂C(o)-.

The term "alkyl" shall refer to any straight-chain or branched carbon group, such as methyl, ethyl, isopropyl, n-propyl, 2-butyl, isobutyl, n-butyl, pentyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1-ethyl-1-methylpropyl, or 1-ethyl-2-methylpropyl. The term "alkyl" shall also include any group which comprises an alkyl group, as named above. Alkyl shall preferably refer to a carbon group having - unless otherwise stated - 1 to 8, in particular 1 to 6, and more preferably 1 to 4 carbon atoms. In some embodiments, methyl is preferred.

The term "cycloalkyl" shall refer to any cycloaliphatic radical. As an example, a cycloalkyl group has from 3 to 8 carbon atoms. In particular, the term cycloalkyl shall refer to a cycloalkyl group having from 3 to 6 carbon atoms, such as cyclopropyl, cyclobutyl, cyclopentyl, or cyclohexyl. The term "cycloalkyl" shall comprise a cyclic structure that is unsubstituted, or substituted, such as a cyclic structure that carries 1, 2, 3 or 4 alkyl radicals, or one or more methyl radicals.

The term "haloalkyl" shall refer to any halogenated alkyl group, such as an alkyl group as named above, which is partially or completely substituted with fluoro, chloro, bromo and/or iodo, e.g. CH₂F, CHF₂, CF₃, CH₂Cl, 2-fluoroethyl, 2-chloroethyl, 2,2,2-trifluoroethyl, or the like. As such, at least 1, or 2, or 3, or 4, or all of the hydrogen atoms of an alkyl group can be replaced by, e.g., 1, 2, 3, 4 or the corresponding number of halogen atoms. If at least 2, or 3, or 4, or any number of the hydrogen atoms are replaced by, e.g., 2, 3, 4 or the corresponding number of halogen atoms, the corresponding halogen atoms can be identical or different halogen atoms. Without being bound thereto, examples of haloalkyl groups are chloromethyl, fluoromethyl, bromomethyl, dichloromethyl, difluoroethyl, trichloromethyl, and the like.

The term "alkenyl" shall refer to any singly unsaturated hydrocarbon radical. Without being bound thereto, the term alkenyl shall include C₂-C₆-alkenyl groups, such as 1-propen-1-yl, allyl (2-propen-1-yl), 2-propen-2-yl, methallyl(2-methylprop-2-en-1-yl), or vinyl, and C₃-C₅-alkenyl groups, such as allyl (2-propen-1-yl), 1-methylprop-2-en-1-yl, 2-buten-1-yl, 3-buten-1-yl, 2-penten-1-yl, 3-penten-1-yl, 4-penten-1-yl, 1-methylbut-2-en-1-yl, 2-ethylprop-2-en-1-yl, or 2-hexen-1-yl.

The term "alkylene" shall refer to any straight-chain or branched alkylene group, preferably having - unless otherwise stated - 1 to 8, in particular 1 to 6, and more preferably 1 to 4 carbon atoms. Without being bound thereto, examples of the term alkylene shall include ethylene, methylene, propylene, butylene, isobutylene, isopropylehtylene, and 1-methylethylene.

According to this invention, the term "heteroatom" shall refer to an atom selected from oxygen, nitrogen and sulfur. According to this invention, any group containing heteroatoms may contain either 1, or 2, or 3 heteroatoms selected from oxygen, nitrogen and sulfur. The 1, or 2, or 3 heteroatoms may be either the same or different.

The term "heteroalkylene" shall refer to any straight-chain or branched alkyl group, which comprises between 1 and 3 heteroatoms. Without being bound thereto, examples of the term heteroalkylene shall include alkylaminoalkyl, dialkylaminoalkyl, and alkyloxyalkyl.

The term "aryl" refers to any aromatic cyclic radical having between 6 and 12 atoms. The term aryl can refer to a monocyclic aromatic ring, such as phenyl, or to a fused polycyclic aromatic ring comprising a first monocyclic aromatic ring and one or more carbocycles which are saturated, partially unsaturated or aromatic, such as indenyl or naphthyl.

The term "alkenylene" shall refer to any straight-chain or branched alkenylene group, such as one comprising between 2 and 4 carbon atoms. Without being bound thereto, examples of the term alkenylene shall include vinyl and propenyl.

The term "alkinylene" shall refer to any straight-chain or branched alkinylene group, such as one comprising between 2 to 4 carbon atoms, for example propynylene.

The term "heteroaromatic" or "heteroaryl" shall refer to any aromatic group having at least one heteroatom as a ring member, i.e an atom selected from oxygen, nitrogen and sulfur. In particular, a heteroaromatic or heteroaryl group comprises at least one nitrogen atom as a ring member. In addition, the heteroaromatic or heteroaryl group can, besides the at least one nitrogen atom, further include an oxygen and/or a sulfur atom as ring member.

The term "bicyclic heteroaromatic group" shall refer to any heteroaromatic ring and an additional anellated, saturated or unsaturated, or aromatic carbocycle, such as a benzene, cyclohexene, or cyclohexadiene ring. Without being bound thereto, examples of the term bicyclic heteroaromatic group shall include quinolinyl, indazolyl, benzofuryl, benzthienyl, isoindolyl, azaindole, dihydroquinolinyl, dihydroisoquinolinyl, dihydrobenzofuryl, chromenyl, chromanyl, tetrahydrobenzothiazolyl benzoxazolyl, benzthiazolyl, benzimidazolyl, imidazo[b]thiazolyl, imidazo[a]pyridyl, pyrazo[a]pyridyl, and pyrrol[d]pyrimidyl.

Without being bound thereto, examples of anellated cycloalkenyl rings include dihydroindolyl, dihydroindolizinyl, dihydroisoindolyl, dihydroquinolinyl, dihydroisoquinolinyl, dihydrobenzofuryl, chromenyl, chromanyl, and tetrahydrobenzothiazolyl.

A heterocyclic group includes at least one heteroatom, i.e. an atom selected from oxygen, nitrogen and sulfur, and may be saturated or partially unsaturated. Without being bound thereto, examples of heteroaromatic, heteroaryl, or heterocyclic groups of a compound according to the present invention, shall include C-bound, 5-membered, saturated rings, such as pyrazol-3-yl, pyrazol-4-yl, pyrazol-5-yl, isoxazol-3-yl, isoxazol-4-yl, isoxazol-5-yl, thiazol-2-yl, isothiazol-4-yl, isothiazol-5-yl, imidazol-2-yl, imidazol-4-yl, imidazol-5-yl, 2-furyl, 3-furyl, 5-furyl, 2-thienyl, 3-thienyl, 5-thienyl, oxazol-2-yl, tetrahydrofuran-2-yl, tetrahydrofuran-3-yl, tetrahydrothien-2-yl, pyrrol-2-yl, pyrrol-5-yl, tetrahydropyrrol-2-yl, tetrahydropyrrol-3-yl, tetrahydropyrazol-3-yl, tetrahydro-pyrazol-4-yl, tetrahydroisoxazol-3-yl, tetrahydroisoxazol-4-yl, tetrahydroisoxazol-5-yl, 1,2-oxathiolan-3-yl, 1,2-oxathiolan-4-yl, 1,2-oxathiolan-5-yl, tetrahydroisothiazol-3-yl, tetrahydroisothiazol-4-yl, tetrahydroisothiazol-5-yl, 1,2-dithiolan-3-yl, 1,2-dithiolan-4-yl, tetrahydroimidazol-2-yl, tetrahydroimidazol-4-yl, tetrahydrooxazol-2-yl, tetrahydrooxazol-4-yl, tetrahydrooxazol-5-yl, tetrahydrothiazol-2-yl, tetrahydrothiazol-4-yl, tetrahydrothiazol-5-yl, 1,3-dioxolan-2-yl, 1,3- dioxolan-4-yl, 1,3-oxathiolan-2-yl, 1,3-oxathiolan-4-yl, 1,3-dithiolan-4-yl, or 1,3,2-dioxathiolan-4-yl; C-bound, 6-membered, saturated rings, such as pyridin-2-yl, pyridin-5-yl, pyridazin-3-yl, pyridazin-4-yl, pyridazin-6-yl, pyrimidin-2-yl, pyrimidin-4-yl, pyrimidin-5-yl, tetrahydropyran-2-yl, tetrahydropyran-3-yl, tetrahydropyran-4-yl, 1,3-dioxan-2-yl, 1,3-dioxan-4-yl, 1,3-dioxan-5-yl, 1,4-dioxan-2-yl, 1,3-dithian-2-yl, 1,3-dithian-4-yl, 1,3-dithian-5-yl, 1,4-dithian-2-yl, piperidin-2-yl, piperidin-3-yl, piperidin-4-yl, tetrahydrothiopyran-2-yl, tetrahydrothiopyran-3-yl, tetrahydrothiopyran-4-yl, 1,3-oxathian-2-yl, 1,3-oxathian-4-yl, hexahydropyrimidin-2-yl, hexahydropyridazin-3-yl, hexahydropyridazin-4-yl, tetrahydro-1,3-oxazin-2-yl, tetrahydro-1,3-oxazin-4-yl, tetrahydro-1,3-oxazin-5-yl, tetrahydro-1,3-oxazin-6-yl, tetrahydro-1,3-thiazin-2-yl, tetrahydro-1,3-thiazin-4-yl, tetrahydro-1,3-thiazin-5-yl, tetrahydro-1,3-thiazin-6-yl, tetrahydro-1,4-thiazin-2-yl, tetrahydro-1,4-thiazin-3-yl, tetrahydro-1,4-oxazin-2-yl, or tetrahydro-1,2-oxazin-6-yl; N-bound, 5-membered, saturated rings, such as tetrahydropyrrol-1-yl, tetrahydroisoxazol-2-yl, tetrahydroisothiazol-2-yl, tetrahydroimidazol-1-yl, tetrahydrooxazol-3-yl, or tetrahydrothiazol-3-yl; N-bound, 6-membered, saturated rings, such as piperidin-1-yl, hexahydropyrimidin-1-yl, pyridazin-6-yl, pyrimidin-2-yl, pyrazin-5-yl, hexahydropyrazin-1-yl, hexahydro- pyridazin-1-yl, or tetrahydro-1,4-oxazin-4-yl; C-bound, 5-membered, partially unsaturated rings, such as 2,3-dihydrofuran-3-yl, 2,5-dihydrofuran-2-yl, 2,5-di-hydrofuran-3-yl, 4,5- dihydrofuran-2-yl, 4,5-dihydrofuran-3-yl, 2,3-dihydro-thien-2-yl, 2,3-dihydrothien-3-yl, 2,5-dihydrothien-3-yl, 4,5-dihydrothien-2-yl, 4,5-dihydrothien-3-yl, 2,3-dihydro-1 H-pyrrol-2-yl, 2,3-dihydro-1 H-pyrrol-3-yl, 2,5-dihydro-1 H-pyrrol-2-yl, 2,5-dihydro-1 H-pyrrol-3-yl, 4,5-dihydro-1 H-pyrrol-2-yl, 4,5-dihydro-1 H-pyrrol-3-yl, 3,4-dihydro-2H-pyrrol-2-yl, 3,4-dihydro-2H-pyrrol-3-yl, 3,4-dihydro-5H-pyrrol-2-yl, 3,4-dihydro-sH-pyrrol-3-yl, 4,5-dihydro-1 H-pyrazol-3-yl, 4,5-dihydro-1 H-pyrazol-4-yl, 4,5-dihydro-1 H-pyrazol-5-yl, 4,5-dihydroisoxazol-4-yl, 4,5-dihydroisothiazol-4-yl, 4,5- dihydroisothiazol-5-yl, 4,5-dihydro-1 H-imidazol-4-yl, 4,5- dihydro-1 H-imidazol-5-yl, 2,5-dihydro-1 H-imidazol-2-yl, 2,5-dihydro-1 H-imidazol-4-yl, 2,5-dihydro-1 H-imidazol-5-yl, 2,3-dihydro-1 H-imidazol-2-yl, 2,3-dihydro-1 H-imidazol-4-yl, 4,5-dihydro-oxazol-2-yl, 4,5-dihydrooxazol-4-yl, 4,5-dihydrooxazol-5-yl, 2,5-dihydrooxazol-2-yl, 2,5-dihydrooxazol-4-yl, 2,5-dihydrooxazol-5-yl, 2,3-dihydrooxazol-2-yl, 4,5-dihydrothiazol-2-yl, 2,5-dihydrothiazol-2-yl, 2,5-dihydrothiazol-4-yl, 2,5-dihydrothiazol-5-yl, 2,3-dihydrothiazol-2-yl, 2,3-dihydrothiazol-4-yl, 2,3-dihydrothiazol-5-yl, 1,3-dioxol-2-yl, or 1,3-dioxol-4-yl; C-bound, 6-membered, partially unsaturated rings, such as 2H-3,4-dihydropyran-6-yl, 2H-3,4-dihydropyran-5-yl, 2H-3,4-dihydrothiopyran-2-yl, 1,2,3,4-tetrahydropyridin-6-yl, 1,2,3,4- tetrahydropyridin-4-yl, 1,2,3,4-tetra-hydropyridin-3-yl, 2H-5,6-dihydropyran-2-yl, 2H-5,6-dihydrothiopyran-2-yl, 2H-5,6-dihydrothiopyran-3-yl, 2H-5,6-dihydrothiopyran-6-yl, 1,2,5,6-tetrahydropyridin-2-yl, 1,2,5,6-tetrahydropyridin-3-yl, 1,2,5,6-tetrahydropyridin-4-yl, 1,2,5,6-tetrahydropyridin-5-yl, 1,2,5,6-tetrahydropyridin-6-yl, 2,3,4,5-tetrahydropyridin-4-yl, 2,3,4,5-tetrahydropyridin-5-yl, 2,3,4,5-tetrahydropyridin-6-yl, 4H-pyran-2-yl, 4H-pyran-3-yl, 4H-pyran-4-yl, 4H-thiopyran-2-yl, 4H-thiopyran-3-yl, 4H-thiopyran-4-yl, 1,4-dihydropyridin-4-yl, 2H-pyran-2-yl, 2H-pyran-3-yl, 2H-pyran-4-yl, 2H-pyran-5-yl, 2H-pyran-6-yl, 2H-thiopyran-2-yl, 2H-thiopyran-3-yl, 2H-thiopyran-4-yl, 2H-thiopyran-5-yl, 1,2-dihydropyridin-2-yl, 1,2-dihydro-pyridin-3-yl, 1,2-dihydropyridin-4-yl, 1,2-dihydropyridin-5-yl, 1,2-dihydro-pyridin-6-yl, 3,4-dihydropyridin-5-yl, 3,4-dihydropyridin-6-yl, 2,5-dihydropyridin-2-yl, 2,5-dihydropyridin-3-yl, 2,5-dihydropyridin-4-yl, 2,5-dihydropyridin-5-yl, 2,5-dihydropyridin-6-yl, 2,3-dihydropyridin-2-yl, 2,3-dihydropyridin-4-yl, 2,3-dihydro-pyridin-5-yl, 2,3-dihydropyridin-6-yl, 2H-5,6-dihydro-1,2-oxazin-3-yl, 2H-5,6-dihydro-1,2-oxazin-5-yl, 2H-5,6-dihydro-1,2-oxazin-6-yl, 2H-5,6-dihydro- 1,2-thiazin-3-yl, 2H-5,6-dihydro-1,2-thiazin-4-yl, 2H-5,6-dihydro-1,2-thiazin-5-yl, 4H-5,6-dihydro-1,2-oxazin-5-yl, 4H-5,6-dihydro-1,2-thiazin-3-yl, 4H-5,6-dihydro-1,2-thiazin-4-yl, 4H-5,6-dihydro-1,2-thiazin-5-yl, 4H-5,6-dihydro-1,2-thiazin-6-yl, 2H-3,6-dihydro-1,2-oxazin-3-yl, 2H-3,6-dihydro-1,2-oxazin-4-yl, 2H-3,6-dihydro-1,2-oxazin-5-yl, 2H-3,6-dihydro-1,2-oxazin-6-yl, 2H-3,6-dihydro-1,2-thiazin-3-yl, 2H-3,6-dihydro-1,2-thiazin-4-yl, 2H-3,4-dihydro-1,2-oxazin-3-yl, 2H-3,4-dihydro-1,2-oxazin-4-yl, 2H-3,4-dihydro-1,2-oxazin-5-yl, 2H-3,4-dihydro-1,2-oxazin-6-yl, 2H-3,4-dihydro-1,2-thiazin-3-yl, 2H-3,4-dihydro-1,2-thiazin-4-yl, 2H-3,4-dihydro-1,2-thiazin-5-yl, 2H-3,4-dihydro-1,2-thiazin-6-yl, 2,3,4,5-tetrahydropyridazin-3-yl, 1,2,5,6-tetrahydropyridazin-4-yl, 1,2,5,6-tetra-hydropyridazin-5-yl, 1,2,5,6-tetrahydropyridazin-6-yl, 4H-5,6-dihydro-1,3-oxazin-2-yl, 4H-5,6-dihydro-1,3-oxazin-6-yl, 4H-5,6-dihydro-1,3-thiazin-2-yl, 4H-5,6-dihydro-1,3-thiazin-4-yl, 4H-5,6-dihydro-1,3-thiazin-5-yl, 4H-5,6-dihydro-1,3-thiazin-6-yl, 3,4,5,6-tetrahydropyrimidin-6-yl, 1,2,3,4-tetrahydropyrazin-2-yl, 1,2,3,4-tetrahydropyrimidin-4-yl, 1,2,3,4-tetrahydropyrimidin-6-yl, 2,3-dihydro-1,4-thiazin-2-yl, 2,3-dihydro-1,4-thiazin-3-yl, 2,3-dihydro-1,4-thiazin-5-yl, 2,3-dihydro-1,4-thiazin-6-yl, 2H-1,3-oxazin-2-yl, 2H-1,3-oxazin-5-yl, 2H-1,3-oxazin-6-yl, 2H-1,3- thiazin-2-yl, 2H-1,3-thiazin-4-yl, 2H-1,3-thiazin-5-yl, 2H-1,3-thiazin-6-yl, 4H-1,3-oxazin-2-yl, 4H-1,3-oxazin-4-yl, 4H-1,3-oxazin-5-yl, 4H-1,3-oxazin-6-yl, 4H-1,3-thiazin-2-yl, 4H-1,3-thiazin-4-yl, 4H-1,3-thiazin-5-yl, 4H-1,3-thiazin-6-yl, 6H-1,3-oxazin-2-yl, 6H-1,3-thiazin-2-yl, 6H-1,3-oxazin-5-yl, 6H-1,3-thiazin-6-yl, 2H-1,4-oxazin-2-yl, 2H-1,4-oxazin-3-yl, 2H-1,4-oxazin-5-yl, 2H-1,4-oxazin-6-yl, 2H-1,4-thiazin-2-yl, 2H-1,4-thiazin-3-yl, 4H-1,4-oxazin-2-yl, 4H-1,4-oxazin-3-yl, 4H-1,4-thiazin-2-yl, 4H-1,4-thiazin-3-yl, 1,4-dihydropyridazin-3-yl, 1,4-dihydropyridazin-4-yl, 1,4-dihydropyrimidin-2-yl, 3,4-dihydropyrimidin-4-yl, or 3,4-dihydropyrimidin-5-yl; N-bound, 5-membered, partially unsaturated rings, such as pyrrol-1-yl, pyrazol-1-yl, imidazol-1-yl, 1,2,3-triazol-1-yl, 2,5-dihydro-1 H-pyrrol-1-yl, 4,5-dihydro-1 H-pyrazol-1-yl, 2,3-dihydro-1 H-pyrazol-1-yl, 2,5-dihydroisoxazol-2-yl, 2,5-dihydroisothiazol-2-yl, 2,3-dihydrooxazol-3-yl, or 2,3-dihydrothiazol-3-yl; and N-bound, 6-membered, partially unsaturated rings, such as 1,2,3,4-tetrahydropyridin-1-yl, 1,2,5,6-tetrahydropyridin-1-yl, 2H-5,6-dihydro-1,2-oxazin-2-yl, 2H-5,6-dihydro-1,2-thiazin-2-yl, 2H-3,4-dihydro-1,2-oxazin-2-yl, 2H-3,4-dihydro-1,2-thiazin-2-yl, 2,3,4,5-tetrahydropyridazin-2-yl, 1,2,3,6-tetrahydropyridazin-1-yl, 1,2,3,4-tetrahydropyrimidin-1-yl, 2,3-dihdro-1,4-thiazin-4-yl, 2H-1,2-oxazin-2-yl, 4H-1,4-thiazin-4-yl, 1,4-dihydropyridazin-1-yl, 1,4-dihydropyrazin-1-yl, 1,2-dihydropyrazin-1-yl, 1,4-dihydropyrimidin-1-yl, or 3,4-dihydropyrimidin-3-yl.

A further preferred embodiment relates to the compound according to this invention, and the pharmaceutically acceptable salts, solvates and optical isomers thereof, wherein 1-(4-((4-((5-cyclopentyl-1H-pyrazol-3-yl)amino)pyrimidin-2-yl)amino)pheny1)-3-(3-(trifluoromethyl)phenyl)urea is excluded.

Yet another particularly preferred embodiment relates to a compound according to this invention, wherein the compound is capable of binding, preferably of specifically binding, to a serine/threonine kinase. According to this invention, the compound is capable of binding, preferably of specifically binding, to any serine/threonine kinase. A particularly preferred serine/threonine kinase, in the context of this invention, is Aurora Kinase A (AURKA), or a variant thereof.

A further preferred embodiment relates to a compound according to this invention, wherein the compound modulates, stabilizes and/or destabilizes, preferably specifically modulates, stabilizes and/or destabilizes, the conformation of the serine/threonine kinase, preferably of AURKA, or the variant thereof.

Additionally preferred is a compound, wherein the compound modulates, mimics, stabilizes and/or destabilizes, preferably specifically modulates, mimics, stabilizes and/or destabilizes, an interaction of the serine/threonine kinase, preferably of AURKA, or the variant thereof, with at least one binding protein.

The expression "modulates" as used herein means that upon administration of a compound according to this invention, the conformation of AURKA is altered, preferably because a binding of the compound to AURKA is inducing a conformational shift in AURKA. Thereby, the compound is modulating, i.e. altering, the binding of at least one binding protein, such as MYC and/or TPX2, to AURKA. Accordingly, the term "modulates" further refers to inducing a shift in the protein-protein interaction (PPI) of AURKA with at least one binding protein, such as a change of the binding of MYC and/or TPX2 to AURKA.

In a particularly preferred embodiment, the compound according to this invention alters and/or modulates the conformation of AURKA, preferably because a binding of the compound to AURKA is inducing a conformational shift in AURKA, and thereby an increased and/or reduced binding of at least one binding protein to AURKA can be observed. Thus, in one embodiment a compound is preferred that alters the conformation of AURKA, thereby modulating, increasing, and/or reducing a binding of at least one binding protein to AURKA. In a further preferred embodiment, said at least one binding protein is MYC and/or TPX2. Yet another preferred embodiment relates to a compound according to this invention that induces a conformational shift in AURKA, thereby modulating the interactome of AURKA.

The term "mimics" as used herein means that a compound according to this invention binds to a location of the serine/threonine kinase, preferably AURKA, which can be identical to or close to a binding region of a particular binding protein. Accordingly, the respective compound of this invention competes with the particular binding protein for binding to AURKA. The particular compound mimicking the binding of a binding protein might have structural features that are similar to those present in the particular binding protein. A compound mimicking a particular binding protein might induce a conformational shift of the serine/threonine kinase, preferably of AURKA, which is comparable to the conformational shift of the serine/threonine kinase, preferably of AURKA, that is induced upon binding of the particular binding protein to the serine/threonine kinase, preferably to AURKA.

The expression "stabilizes" as used herein means that upon administration of a compound according to this invention, a certain conformational state of the serine/threonine kinase, preferably of AURKA, is more likely kept in that particular state than altered into a different conformational state, i.e. the likelihood of a conformational shift of the serine/threonine kinase, preferably of AURKA, is reduced or prevented. The term "stabilizes" can also refer to locking a serine/threonine kinase, preferably AURKA, into a particular conformation. The expression "stabilizes" as used herein further refers to inducing an enhanced binding of certain binding proteins to the serine/threonine kinase, preferably to AURKA. For example, the compounds of this invention stabilize the binding of TPX2 to AURKA.

The expression "destabilizes" as used herein means that upon administration of a compound according to this invention, a certain conformational state of the serine/threonine kinase, preferably of AURKA, is more likely altered into a different conformational state than kept or locked in that particular conformational state, i.e. the likelihood of a conformational shift of the serine/threonine kinase, preferably of AURKA, is enhanced or enforced. The term "destabilizes" can also refer to inducing a conformational shift in the serine/threonine kinase, preferably of AURKA. The expression "destabilizes" as used herein further means that the induction of a conformational shift in the serine/threonine kinase, preferably AURKA, upon administration of a compound according to this invention, induces an alteration and/or modulation of the AURKA interactome. Accordingly, the term "destabilizes" further refers to inducing a reduced binding of certain binding proteins to the serine/threonine kinase, preferably to AURKA. For example, the compounds of this invention destabilize the binding of MYC to AURKA.

Yet another preferred embodiment relates to a compound according to this invention, wherein the modulating, mimicking, stabilizing and/or destabilizing, preferably the specifically modulating, mimicking, stabilizing and/or destabilizing, of the interaction of the serine/threonine kinase, preferably AURKA, or a variant thereof, with the at least one binding protein in a cell, induces the death of at least one cell, preferably wherein the cell is a tumor cell and/or a diseased cell. More preferably, the death of the at least one cell, preferably the tumor cell and/or the diseased cell occurs upon administration of a compound according to this invention to a subject, such as a patient.

In this context, the experimental determination and quantification of cell death will be performed using standard methods, which the person of skill is well aware of. For example, the number of dead cells can be quantified using immunohistochemical methods, such as a TUNEL staining, and/or a staining for ANNEXIN V/PI. However, any method to detect and/or quantify dead cells can be used.

According to this invention, upon administration of a compound according to any of the particular embodiments as disclosed herein, any protein of the AURKA interactome may be influenced. In a particularly preferred embodiment, the at least one binding protein is TPX2 and/or MYC.

Further preferred is a compound, wherein the compound modulates, mimics, stabilizes and/or destabilizes, preferably specifically modulates, mimics, stabilizes and/or destabilizes, an interaction of a serine/threonine kinase, wherein the serine/threonine kinase is AURKA, or a variant thereof, with at least one binding protein selected from TPX2 and MYC, and wherein the compound stabilizes and/or increases, preferably specifically stabilizes and/or increases, the interaction of AURKA, or the variant thereof, with TPX2, and/or wherein the compound destabilizes and/or decreases, preferably specifically destabilizes and/or decreases, the interaction of AURKA, or the variant thereof, with MYC.

The present invention is based on the surprising finding that the compounds of this invention are modulators of the conformation of AURKA, thereby modulating the binding of certain binding proteins to AURKA. Upon administration of the compounds of this invention, a conformational shift in AURKA induces an alteration of the AURKA interactome, and preferably induces an enhanced binding of TPX2 to AURKA and/or a reduced binding of MYC to AURKA.

In this context, the experimental determination and quantification of binding of a protein to AURKA will be performed using standard methods, which the person of skill is well aware of. For example, the binding of a binding protein, such as MYC and/or TPX2, to AURKA, can be determined by Co-Immunoprecipitation (Co-IP), followed by western blotting. As is well aware to the person of skill, Co-IP comprises the pulldown of one protein (e.g., AURKA, or a potential binding protein, such as MYC and/or TPX2), and the subsequent staining for the respective second protein (e.g. the potential binding protein, such as MYC and/or TPX2, in case of an AURKA-pulldown, or AURKA in case of a MYC and/or TPX2 pulldown) in, for example, a western blot of the pulldown eluate. Moreover, the interaction and/or binding of two proteins, such as the interaction and/or binding of MYC and/or TPX2 to AURKA, can also be determined by Proximity Ligation Assay (PLA), where fixed cells are incubated with, for example, MYC and AURKA specific antibodies, or TPX2 and AURKA specific antibodies, wherein the MYC/TPX2 and AURKA specific antibodies are derived from a different species. Then, incubation with two PLA probes, directed against either species of the MYC/TPX2 and AURKA specific antibodies, followed by ligation and amplification, enables visualization of foci that are indicative for a close proximity between MYC/TPX2 and AURKA. Thus, PLA foci can be visualized with a microscope to determine the amount of interaction and/or binding between MYC/TPX2 and AURKA.

It was surprisingly found that the novel compounds of this invention diminish the MYC-AURKA interaction and, simultaneously, enhance the TPX2-AURKA interaction.

Interestingly, the inventors found that a compound comprising an anilide structure according to formula (III), is diminishing the MYC-AURKA interaction. Generally speaking, the binding of MYC to AURKA prevents ubiquitination of MYC, and, thereby, prevents proteasomal degradation of MYC. Accordingly, a compound comprising an anilide structure according to formula (III), is increasing the amount of ubiquitinated MYC, whereby the proteasomal degradation of MYC is enhanced. Therefore, the compounds of this invention are particularly useful in inducing MYC degradation on the cellular level.

The inventors observed that a compound comprising an anilide structure according to formula (III), is reducing the MYC-AURKA interaction, thereby diminishing the stabilization of MYC by AURKA or the variant thereof. The inventors were able to show that the compounds of this invention inhibit the MYC-AURKA interaction on a cellular level specifically and induce MYC degradation rapidly, and yet do not or only insignificantly inhibit the enzymatic activity of AURKA. Hence, the inventors surprisingly demonstrated that it is possible to modify the level of MYC indirectly by targeting the interaction of Myc with AURKA with small molecules via disrupting the AURKA-mediated protection mechanism, while simultaneous inhibition of the enzymatic activity of AURKA is not required. The inventors also surprisingly demonstrated that the compounds of this invention can further target and modulate the interaction of TPX2 with AURKA.

Accordingly, a particularly preferred embodiment relates to a compound according to this invention, wherein the compound destabilizes and/or decreases, preferably specifically destabilizes and/or decreases, the level of MYC in a cell, and/or the activity of MYC in a cell.

Yet another preferred embodiment relates to a compound according to this invention, wherein the compound induces an abnormal and/or defect mitosis of a cell, and wherein the compound induces the formation of at least one abnormal and/or defect spindle in the cell.

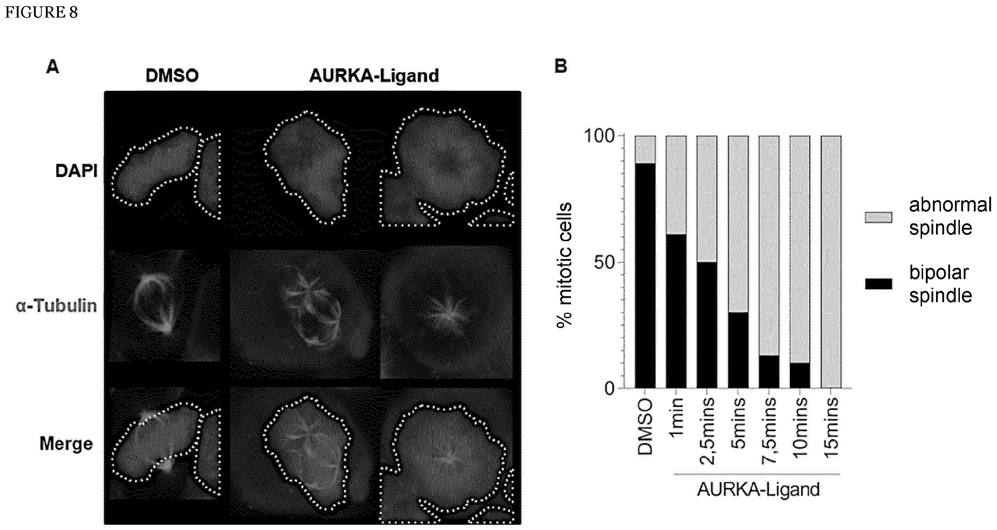

According to this invention, the term "mitosis" as used herein shall refer to the part of the cell cycle when replicated chromosomes are separated into two new nuclei. The term "cell division" shall refer to dividing one cell into two genetically identical cells in which the number of chromosomes is maintained. Importantly, the interaction between AURKA and TPX2 is essential for the formation of the mitotic spindle, as well as the proper separation of the centrosomes after the mitotic spindle has been formed. An enhanced binding of AURKA to TPX2, as occurs in the presence of a compound comprising an anilide structure according to formula (III), is, thereby, inducing an abnormal and/or defect mitosis of a cell, for example by inducing the formation of at least one abnormal and/or defect spindle in the cell. The inventors observed that the compounds of this invention trigger the formation of abnormal (e.g. multipolar and monopolar) spindles a few minutes after application of a compound comprising an anilide structure according to formula (III) (see Figure 8).

The inventors also observed that upon administration of a compound comprising an anilide structure according to formula (III), to a cell or to multiple cells, the cell(s) are (transiently) arrested and/or locked in mitosis, and/or cell(s) are not dividing at a proper and/or a normal rate, when compared to cells not treated with a compound according to this invention. Also, the inventors observed that the administration of a compound comprising an anilide structure according to formula (III), to a cell, leads to cell death. Causative for the enhanced amount of cell death upon administration of a compound comprising an anilide structure according to formula (III), to a cell, is most likely the formation of abnormal spindles in the cell and/or subsequent defects in mitosis and/or cytokinesis of cells treated with a compound comprising an anilide structure according to formula (III), which is triggered by, for example, a modulated AURKA interactome, such as by an enhanced binding of TPX2 to AURKA.

A further preferred embodiment pertains to a compound according to this invention, wherein the compound does not destabilize and/or decrease the binding of ATP to the serine/threonine kinase, preferably to AURKA, or to the variant thereof.

The inventors surprisingly found that the compounds of this invention induce cell death of malignant cells in the low nanomolar range. Importantly, the determined IC50 of the compounds of this invention are comparable or better than the IC50 of alisertib. Importantly, a dependency on an ATP-competitive core in AURKA is not necessary for modulation of the PPI of AURKA with MYC and TPX2 when using the compounds of this invention, and, hence, the compounds of this invention do not inhibit the enzymatic activity of AURKA at concentrations sufficient to target malignant cells. This significantly contrasts the compounds of this invention from compounds like alisertib, which modulate the protein-protein interaction of AURKA with MYC, while simultaneously inhibiting the enzymatic activity of AURKA. Thus, the inventors surprisingly found that the compounds of this invention can influence the AURKA interactome selectively by inducing a conformational change of AURKA at required concentrations that do not affect the kinase activity of AURKA, thereby triggering, e.g., an enhanced binding of TPX2 to AURKA and/or a reduced binding of MYC to AURKA without inhibiting the enzymatic activity of AURKA. Thus, the inventors demonstrated that it is possible to uncouple the kinase inhibition of AURKA from selectively influencing the AURKA interactome. The effects observed upon administration of the compounds of this invention are most likely triggered by at least an induced reduced binding of MYC to AURKA, thereby destabilizing MYC and leading to its degradation, and an enhanced binding of TPX2 to AURKA. The latter is accompanied by observing an abnormal and/or defect mitosis of cells treated with a compound according to this invention compared to cells not treated with a compound according to this invention, and the formation of at least one abnormal and/or defect spindle in those cells treated. The observed effect underlines that a normal interaction between AURKA and TPX2 is essential for the formation of the mitotic spindle, and, accordingly, for mitosis.

Accordingly, the inventors found that the compounds of this invention, i.e. compounds comprising an anilide structure according to formula (III), are not dependent on inhibiting the catalytic activity of AURKA. Instead, the compounds of this invention, i.e. compounds comprising an anilide structure according to formula (III), are modulating the conformation of AURKA, thereby altering the binding of AURKA to MYC and/or TPX2, without (strongly) inhibiting the catalytic activity of AURKA. A particularly preferred compound of this invention is not inhibiting the catalytic activity of AURKA, and yet is modulating the conformation of AURKA, thereby altering the binding of AURKA to MYC and/or TPX2 without influencing the enzymatic activity of AURKA.

It was thus surprisingly found that a compound comprising an anilide structure according to formula (III), is able to overcome the major drawbacks of conventional AURKA inhibitors. This invention, therefore, demonstrates that in contrast to CD532 or alisertib, a dependency on an ATP-competitive core in AURKA is not necessary for modulation of the PPI of AURKA with MYC, and, hence, the compounds of this invention do not inhibit the enzymatic activity of AURKA at concentrations sufficient to target tumor cells. Importantly, the inventors' compounds also have no kinase off-target effects at concentrations required to target tumor cells. Accordingly, the inventors were able to uncouple the kinase inhibition of AURKA from influencing the AURKA interactome, such as from inhibiting the MYC-AURKA interaction, and enhancing the TPX2-AURKA interaction.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

A further embodiment relates to a compound according to this invention, wherein the compound stabilizes and/or increases at least one phosphorylation at an amino acid of the amino acid sequence of AURKA according to SEQ ID No: 1, or of the amino acid sequence of the variant thereof, such as a phosphorylation of the amino acid at location 288 when aligned to the amino acid sequence of SEQ ID NO: 1.

Also preferred is a compound according to this invention, wherein the compound enters a cell by diffusion. In yet another preferred embodiment, the cell is a mammalian cell, such as a human cell, for example a human cancer cell. In a further embodiment, the cell is a recombinant host cell, wherein the recombinant host cell expresses the serine/threonine kinase, preferably AURKA, or the variant thereof, and/or the at least one binding protein, preferably wherein the recombinant host cell is a mammalian cell, a bacterial cell, or a yeast cell.

A further aspect of this invention relates to a pharmaceutical composition comprising a compound according to this invention, and a pharmaceutically acceptable carrier, stabilizer and/or excipient. Pharmaceutical excipients that may be added include, without being limited thereto, preservatives, antioxidants, and antimicrobial agents; wetting, emulsifying, and suspending agents; fragrance and colouring additives; compositions for improving compressibility; compositions for creating a delayed, sustained or controlled release of the active ingredient; and various salts to change the osmotic pressure of the pharmaceutical preparation, or to act as a buffer. Such excipients are well known to the skilled artisan.

The compounds of this invention are customarily administered in the form of pharmaceutical compositions, which comprise at least one compound comprising an anilide moiety according to formula (III), optionally together with an inert carrier (e.g. a pharmaceutically acceptable excipient) and, where appropriate, other compounds and/or drugs.

The skilled artisan is aware of suitable forms of pharmaceutical compositions, for example solid forms, such as powders, granules, tablets, in particular film tablets, lozenges, sachets, cachets, sugar-coated tablets, capsules, such as hard gelatin capsules and soft gelatin capsules, or suppositories, semisolid medicinal forms, such as ointments, creams, hydrogels, pastes or plasters, or liquid medicinal forms, such as solutions, emulsions, in particular oil-in- water emulsions, suspensions, for example lotions, injection preparations and infusion preparations. In addition, it is also possible to use liposomes or microspheres.

In a particularly preferred embodiment, a pharmaceutical composition comprising at least one compound comprising an anilide moiety according to formula (III), a pharmaceutically acceptable salt, solvate or optical isomer thereof, is provided in form of a gel, an ointment, a salve, a cream, a tablet, a pill, a capsule, a troche, a dragée, a powder, an aerosol spray, a nasal spray, a suppository, and/or a solution.

When producing a pharmaceutical composition according to this invention, at least one compound comprising an anilide moiety according to formula (III), is optionally mixed or diluted with one or more carriers and/or excipients. Carriers and/or excipients, according to this invention, can be solid, semisolid or liquid materials, which may serve as a vehicle, a carrier or a medium for the active compound.

Further, a pharmaceutical composition according to this invention might further comprise at least one acceptable auxiliary substance, such as a wetting agent, an emulsifying agent, a suspending agent, a preservative, an antioxidant, an antiirritant, a chelating agent, a coating agent, an emulsion stabilizer, a gel former, a resin, a solvent, a solubilizer, a neutralizing agent, a pigment, a silicone derivative, a binder, a filler, a drying agent, a thickener, a wax, a plasticizer, or the like.

A further aspect of this invention pertains to a compound according to this invention, or a pharmaceutical composition comprising a compound according to this invention, for use in medicine.

Yet another aspect of this invention relates to a compound according to this invention, or a pharmaceutical composition comprising a compound according to this invention, for use in the prevention and/or treatment of a proliferative disease, such as cancer, preferably wherein the cancer is a solid cancer, such as a cancer stemming from any solid organ tissue, or a liquid cancer.

The term "treatment" shall refer to a partial or total inhibition and/or reduction of symptoms of a particular disease, such as a proliferative disease, like cancer, in a subject, as well as a partial or total destruction of, for example, diseased cells, or cancer cells. The term "prevention" shall refer to preventing or delaying the onset of a clinically evident disease, such as a proliferative disease, like cancer, in a subject. In the context of the present invention, prevention and/or treatment shall include both preventive and/or actual treatment of the disease symptoms of a disease, such as a proliferative disease, like cancer, which can be alleviated and/or even completely removed using said treatment.

A "proliferative disease" in the context of the present invention shall preferably refer to a disease such as a cancer or a tumor disease. Cancer diseases or tumor diseases that can be treated by a compound or pharmaceutical composition of the present invention include, but are not limited to, cancer or tumor diseases selected from the group of liver cancer, hepatocellular carcinoma (HCC), lung cancer, small cell lung cancer (SCLC), bladder cancer, ovarian cancer, uterine cancer, endometrial cancer, breast cancer, gastric cancer, urinary bladder cancer, renal cancer, pancreatic cancer, stomach cancer, cervical cancer, cephalic cancer, thyroid cancer, esophageal cancer, medulloblastoma, head and neck cancer, lymphoma, leukemia, acute myeloid leukemia, acute lymphocytic leukemia, chronic lymphocytic leukemia, chronic myeloid cancer, prostate cancer, salivary gland cancer, bone cancer, brain cancer, glioma, colon cancer, rectal cancer, colorectal cancer, kidney cancer, skin cancer, melanoma, glioblastoma, squamous cell carcinoma, pleomorphic adenoma, endometrioma, nasopharynx cancer, small intestine cancer, testicular cancer, Hodgkin lymphoma, non-Hodgkin lymphoma, cancer of the anus, cancer of the anal canal, cancer of the anorectum, cancer of the oropharynx, vaginal carcinoma, vulval carcinoma, enteric cancer, endocrine gland cancer, adrenal cancer, urethral cancer, lymphocytoma, cystic cancer, nephritic cancer, hydrouretic cancer, renal cell carcinoma, renal pelvic carcinoma, hypophyseal adenomatosis, adenocarcinoma, and/or a MYC-dependent tumor. Preferred cancers are liver cancer, more preferably hepatocellular carcinoma (HCC), even more preferably *TP53* deficient or mutant HCC, and lung cancer, in particular small cell lung cancer (SCLC), preferably *TP53*/*RB1* mutant and/or deficient SCLC.

Yet another preferred embodiment relates to the compound for use in the treatment and/or prevention of a proliferative disease in a subject, wherein the proliferative disease is a cancer which is refractory or resistant to treatment with at least one prior therapy.

For the prevention and/or treatment of a disease according to this invention, the appropriate dosage of a compound according to this invention will depend on a multitude of different variables, such as the specific type of disease, in particular the specific type of the cancer disease to be treated, the severity and course of the disease, in particular the severity and course of the specific cancer disease, previous therapy, the patient's clinical history, age, size/weight, sex, and response to the compound according to this invention.

A particularly preferred embodiment relates to a compound or a pharmaceutical composition comprising a compound as above for use according to this invention, wherein the compound is modulating, mimicking, stabilizing and/or destabilizing, preferably specifically modulating, mimicking, stabilizing and/or destabilizing, an interaction of a serine/threonine kinase, preferably AURKA, or a variant thereof, with at least one binding protein in a cell, such as in a human cancer cell, preferably wherein the serine/threonine kinase is AURKA, or a variant thereof, and the at least one binding protein is MYC, and/or TPX2, thereby preventing and/or treating the proliferative disease, such as cancer, in the subject.

Further preferred is that the modulating, mimicking, stabilizing and/or destabilizing, preferably specifically modulating, mimicking, stabilizing and/or destabilizing, of an interaction of a serine/threonine kinase, preferably AURKA, or a variant thereof, with at least one binding protein in a cell, induces the death of at least one tumor cell. Also preferred is a compound according to this invention, or a pharmaceutical composition comprising a compound according to this invention, wherein the compound enters a cell by diffusion, and then modulates, mimics, stabilizes and/or destabilizes, preferably specifically modulates, mimics, stabilizes and/or destabilizes, the interaction of a serine/threonine kinase, preferably AURKA, or a variant thereof, with the at least one binding protein in the cell. Preferably, said modulating, mimicking, stabilizing and/or destabilizing, preferably specifically modulating, mimicking, stabilizing and/or destabilizing, of the interaction of the serine/threonine kinase, preferably AURKA, or the variant thereof, then induces the death of at least one tumor cell.

In an additionally preferred embodiment, the subject to be treated is a mammal, such as a mouse, rat, guinea pig, rabbit, cat, dog, monkey, or a human, preferably a human, for example a human patient, more preferably a human patient suffering from a proliferative disease, such as cancer. As will be further described herein, the subject or patient subjected to the treatment or uses of the invention in preferred embodiments is suffering from a proliferative disease, such as cancer. In a further preferred embodiment, the subject is suffering from a recurrent cancer disease.

Yet another embodiment relates to a subject, wherein the subject is at risk of having a proliferative disease, such as cancer. As used herein, the term "being at risk" refers to having a risk that is higher, preferably significantly higher, of developing a proliferative disease, such as cancer, compared to the majority of a matched group defined by basic medical factors such as age, gender, weight, and so on, which is well-known to the skilled person. The subject may be at risk due to a genetic predisposition, or due to exposure to carcinogenic agents, such as ionizing radiation or chemical mutagens. In case of lung cancer, a subject may also be at risk due to cigarette smoking.

Yet another aspect of this invention relates to a method for treating and/or preventing a proliferative disease, such as cancer, in a subject, comprising administering to the subject a therapeutically effective amount of a compound, and/or of a pharmaceutical composition according to this invention. The cancer to be treated and/or prevented is preferably a cancer as defined herein above.

As used herein, the term "therapeutically effective amount" shall refer to the amount of a compound or a combination of different compounds that will elicit the biological or medical response of a tissue, system, animal or human subject that is being sought, for instance, by a researcher or clinician, in accordance with the herein disclosed invention. Furthermore, the term "therapeutically effective amount" means any amount which, as compared to a corresponding subject who has not received such an amount of a compound or a combination of different compounds according to this invention, results in improved treatment, healing, prevention, or amelioration of a disease, disorder, or side effect, or a decrease in the rate of advancement of a disease or disorder.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from cell culture assays and animal studies can be used for formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of this invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The compounds and/or pharmaceutical compositions of this invention can be included in a container, pack, or dispenser, together with instructions for administration.

Yet another aspect of this invention, which can be combined with each embodiment and aspect of this invention, relates to a method of treatment and/or prevention of a proliferative disease in a subject, comprising administering to the subject a therapeutically effective amount of a compound according to this invention, or of a pharmaceutical composition comprising a compound according to this invention. The proliferative disease to be treated and/or prevented with the method of treatment and/or prevention can be any proliferative disease. In a preferred embodiment, the proliferative disease is cancer, preferably wherein the cancer is a solid cancer, such as a cancer stemming from any solid organ tissue, or a liquid cancer.

In accordance with all aspects and embodiments of the medical uses and methods of treatment provided herein, the effective amount of the compounds or combinations of compounds according to this invention administered at least once to a subject in need of treatment, is typically between about 0.01 mg/kg and about 100 mg/kg per administration, such as between about 1 mg/kg and about 10 mg/kg per administration. In some embodiments, the effective amount of a compound according to this invention administered at least once to said subject is between about 0.01 mg/kg and about 0.1 mg/kg per administration, between about 0.1 mg/kg and about 1 mg/kg per administration, between about 1 mg/kg and about 5 mg/kg per administration, between about 5 mg/kg and about 10 mg/kg per administration, between about 10 mg/kg and about 50 mg/kg per administration, or between about 50 mg/kg and about 100 mg/kg per administration.

The amount to be administered can be administered at one time or over a series of treatments. If the effective amount is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

In a preferred embodiment, the compound and/or the pharmaceutical composition is administered systemically and/or locally, preferably wherein the compound and/or the pharmaceutical composition is administered by oral, transdermal (topical), intravenous, vaginal, intranasal, intrathecal, intraarterial, intradermal, subcutaneous, intracerebroventricular, intraparenchymal, intratumoral, transmucosal, rectal, bronchial, and/or parenteral administration, or by any clinically/medically accepted method.

As is well aware to the person of skill, the term systemic administration may comprise injection or infusion into a suitable body tissue or cavity, as well as intraarterial, intradermal, transdermal (such as subcutaneous), intraspinal, intrathecal, intramuscular or intravenous delivery of a pharmaceutical composition containing the compound of this invention in a suitable liquid form, such as an aqueous solution, emulsion or suspension. Also included is ingestion or administration of a pharmaceutical composition containing said compound in a suitable solid or liquid form for transdermal delivery thereof, for instance in form of a transdermal patch or a subepidermal (subcuticular) implant, for peroral delivery thereof.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants, such as ascorbic acid or sodium bisulfate; chelating agents such as ethylenediaminetetraacetic acid; buffers such as acetates, citrates or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions or dispersions and sterile powders for the extemporaneous preparation of sterile injectable solutions or dispersions. For intravenous administration, suitable carriers include physiological saline, bacteriostatic water, or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating, such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying, which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressured container or dispenser which contains a suitable propellant, e.g., a gas, such as carbon dioxide, or a nebulizer.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

In a preferred embodiment, the compound and/or the pharmaceutical composition according to this invention is administered to a subject in an amount expressed as milligrams per kilogram of body weight of said subject, per day, i.e. in form of "mg/kg/day". The expression "per day", as used herein, should not be interpreted as necessarily requiring that any particular dosage form be administered on a daily basis to the subject being treated. The expression "per day" is merely an indication of the smallest convenient but yet arbitrary segment of time, which is being used as part of the overall unit for measuring the dose of an effective compound being administered. Depending on the route of application and other details, which the person of skill is well aware of, the daily dosage may be split into a number of sub-doses for subsequent administration in regular intervals, or, when using sustained or controlled release agents, several daily dosages may be joined into a single depot dosage. The dose, i.e., the therapeutically effective amount of a compound comprising an anilide moiety according to formula (III), a pharmaceutically acceptable salt, solvate or optical isomer thereof, for treating or preventing a proliferative disease, such as cancer, will usually range from about 0.1 mg/kg/day to about 20.0 mg/kg/day, preferably from about 0.1 mg/kg/day to about 12.0 mg/kg/day, more preferably from about 0.5 mg/kg/day to about 10.0 mg/kg/day, and most preferably from about 0.5 mg/kg/day to about 8.0 mg/kg/day. It is necessary for the skilled artisan not only to determine the preferred route of administration and the corresponding dosage form and amount, but said artisan must also determine the dosing regimen, i.e., the frequency of dosing. Particularities of absorption, metabolism and excretion characteristic for the respective tumor type, or found in an individual subject, will have to be taken into account. Most likely, once-a-day (s.i.d.) dosing or twice-a-day (b.i.d.) dosing is preferred.

In another particularly preferred embodiment, the compound and/or the pharmaceutical composition is administered in concentrations that provide in *vivo* concentrations of the compound and/or the pharmaceutical composition in the subject to be treated of between 0.01 mg/kg/day and 1 mg/kg/day.

Further preferred is a method for treating and/or preventing a proliferative disease, such as cancer, in a subject, according to this invention, wherein the compound is administered to the subject in combination with other therapeutics, such as chemotherapeutically active substances. The invention, therefore, further relates to a combination comprising a compound of this invention with one or more further therapeutic agents, such as chemotherapeutically active substances, in particular for use in treating a proliferative disease, such as cancer. Particular agents suitable for combination would be readily apparent to the skilled artisan in the field, and these will usually be determined by the circumstances under which the therapeutic agent and/or compound of the present invention is administered.

It is within the scope of this invention that co-administration of a compound according to this invention, and one or more further therapeutic agents, such as chemotherapeutically active substances, could be by means of the same or of different dosage forms and routes of administration. The present invention further contemplates the use of such combinations in accordance with different dosing schedules. Such combinations to devise and administer would be well within the skill of the artisan.

Examples of such other therapeutics, such as chemotherapeutically active substances, include, but are not limited to, cytostatic agents, fuoropyrimidines, cytotoxic agents, nucleoside analogs (NA), pyrimidine nucleosides, purine nucleosides, alkylating agents, anti-folates, taxanes, platinum agents, vinca alkaloids, anthracyclines/anthracenediones, topoisomerase inhibitors, epipodophyllotoxins, camptothecins, lysyl oxidase homolog 2 (LOXL2) inhibitors, PDE3 inhibitors, PDE4 inhibitors, phospholipase C (PLC) inhibitors, Rho associated protein kinase 2 (ROCK2) inhibitors, hormones, hormonal complexes, antihormonals, antivirals, enzymes, proteins, peptides, polyclonal and/or monoclonal antibodies, epothilones, antimicrotubule agents, and the like. However, any therapeutic agent may be combined with a compound according to this invention to generate a combination as above, wherein said therapeutic agent preferably tackles specifically rapidly dividing cells.

Yet another preferred embodiment relates to a combination therapy as above, wherein the combination partners may be administered simultaneously or consecutively. The term "simultaneous administration" shall refer to a treatment regime, wherein the individual components are administered together, either in the form of a single pharmaceutical composition or a device comprising or containing both components, or as separate compositions or devices, each comprising one of the components, but both are administered simultaneously. Such combinations of the separate individual components for simultaneous combination may be provided in the form of a kit. The term "consecutive administration" or "adjunctive administration" shall refer to a treatment regime, wherein the individual components are administered consecutively, coterminous or overlapping in the form of separate pharmaceutical compositions or devices. This regime of therapeutic administration of two or more therapeutic agents is referred to generally by those skilled in the art as "adjunctive therapeutic administration", and is also known as "add-on therapeutic administration". Such combinations of the separate individual components for adjunctive administration of a combination may be provided in the form of a kit.

Any and all treatment regimes in which a subject receives a separate but overlapping therapeutic administration of a compound according to this invention and at least one further therapeutic agent are within the scope of the current invention. Any combination therapy of the present invention may be administered adjunctively. In a preferred embodiment as described herein, a patient is first stabilized on a therapeutic administration of one or more of the components for a period of time and then receives administration of another component. A certain test might be included between administration of the first component and the second component. Such a test might be beneficial for the clinical outcome of the particular treatment, because first line treatments are often insufficient to destroy all tumor cells from the organism.

The combination of a compound of this invention with one or more further therapeutic agents, such as chemotherapeutically active substances, in particular for use in treating a proliferative disease, such as cancer, can be administered on a regularly scheduled basis. It is also envisioned that administration in combinations could assume a number of different forms and still be within the scope of the present invention.

A compound according to the present invention in combination with one or more further therapeutic agents, such as chemotherapeutically active substances, which are to form the intended combination therapy, should be formulated into a convenient dosage form, such as an oral tablet, containing all of the drugs forming the combination. Varying half-lives for the different drugs can be accommodated by the person skilled in the art when preparing formulations of the combination therapy by creating, for example, controlled-release forms of said compounds and/or therapeutics with different release times so that relatively uniform dosing can be achieved once the combination is administered to the patient. The skilled artisan is familiar with pharmaceutical dosage forms that are useful according to the present invention.

Further preferred is that the subject administered a compound, a combination, and/or a pharmaceutical composition according to this invention is a patient, preferably a cancer patient, more preferably a relapsed or refractory cancer patient, wherein a previous cancer therapy, for example a cancer therapy with chemotherapeutically active substances, has failed in the relapsed or refractory cancer patient.

Cancer diseases that can be treated by the compound, the combination, and/or the pharmaceutical composition of the present invention also include recurrent cancer diseases. The term "recurrent cancer diseases", as disclosed herein, comprises a recurrent cancer selected from the group, but not limited to, liver cancer, hepatocellular carcinoma (HCC), lung cancer, small cell lung cancer (SCLC), bladder cancer, ovarian cancer, uterine cancer, endometrial cancer, breast cancer, gastric cancer, urinary bladder cancer, renal cancer, pancreatic cancer, stomach cancer, cervical cancer, cephalic cancer, thyroid cancer, esophageal cancer, medulloblastoma, head and neck cancer, lymphoma, leukemia, acute myeloid leukemia, acute lymphocytic leukemia, chronic lymphocytic leukemia, chronic myeloid cancer, prostate cancer, salivary gland cancer, bone cancer, brain cancer, glioma, colon cancer, rectal cancer, colorectal cancer, kidney cancer, skin cancer, melanoma, glioblastoma, squamous cell carcinoma, pleomorphic adenoma, endometrioma, nasopharynx cancer, small intestine cancer, testicular cancer, Hodgkin lymphoma, non-Hodgkin lymphoma, cancer of the anus, cancer of the anal canal, cancer of the anorectum, cancer of the oropharynx, vaginal carcinoma, vulval carcinoma, enteric cancer, endocrine gland cancer, adrenal cancer, urethral cancer, lymphocytoma, cystic cancer, nephritic cancer, hydrouretic cancer, renal cell carcinoma, renal pelvic carcinoma, hypophyseal adenomatosis, and/or adenocarcinoma. Preferred recurrent cancers to be treated by the compound of the present invention are recurrent liver cancer, more preferably recurrent hepatocellular carcinoma (HCC), and recurrent lung cancer, in particular recurrent small cell lung cancer (SCLC).

Yet another aspect of this invention pertains to the in vitro use of a compound or a pharmaceutical composition according to this invention, in the manufacture of a medicament against a proliferative disease, such as cancer.

A further aspect of this invention relates to a kit comprising:
i) a compound, a combination of compounds, or a pharmaceutical composition according to this invention, and
ii) written instructions to apply the compound, the combination of compounds, or the pharmaceutical composition,
iii) optionally, a container holding the compound, the combination of compounds, or the pharmaceutical composition, and the written instructions.

Another aspect of this invention pertains to a method for identifying and/or characterizing a compound according to this invention, wherein the compound is suitable for the treatment of a proliferative disease, the method comprising the steps of:
a) Providing an Aurora Kinase A (AURKA) protein, or a variant thereof, an AURKA binding protein, an ATP molecule, and a candidate compound;
b) Bringing into contact the AURKA protein, or the variant thereof, the AURKA binding protein, the ATP molecule, and the candidate compound;
c) Detecting and/or quantifying a binding between the AURKA protein, or the variant thereof, and the AURKA binding protein,
d) Detecting and/or quantifying a binding between the AURKA protein, or the variant thereof, and the ATP molecule,
wherein a differential level of the binding between the AURKA protein, or the variant thereof, and the AURKA binding protein as detected and/or quantified in c) contacted with the candidate compound compared to the binding between the AURKA protein, or the variant thereof, and the AURKA binding protein as detected and/or quantified in c) not contacted with the candidate compound, and no differential level of the binding between the AURKA protein, or the variant thereof, and the ATP molecule as detected and/or quantified in d) contacted with the candidate compound compared to the binding between the AURKA protein, or the variant thereof, and the ATP molecule as detected and/or quantified in d) not contacted with the candidate compound indicates the candidate compound as suitable for the treatment of the proliferative disease.

Also preferred is the above method, further comprising the step of testing the enzymatic activity of AURKA prior determining whether the candidate compound is a suitable compound for the treatment of the proliferative disease.

Another aspect of this invention, which can be combined with every other preferred embodiment and/or aspect of this invention, pertains to a compound or a pharmaceutical composition according to this invention, for use in selectively modulating the interaction of a serine/threonine kinase, preferably AURKA, or a variant thereof, with at least one binding protein, preferably wherein the serine/threonine kinase is AURKA, or the variant thereof, and the at least one binding protein is MYC, and/or TPX2.

Yet another aspect of this invention relates to the compounds according to this invention for use in modulating the AURKA interactome.

A further aspect of this invention, which can be combined with every other preferred embodiment and/or aspect of this invention, relates to a method of selectively modulating the interaction of AURKA, or a variant thereof, with at least one binding protein, wherein said method comprises the step of contacting AURKA, or a variant thereof, with a compound according to this invention, preferably wherein the at least one binding protein is MYC, and/or TPX2.

In a particularly preferred embodiment, the method of selectively modulating the interaction of AURKA, or a variant thereof, with at least one binding protein, is performed in a cell-free system, or in a cell, such as in a biological assay cell or in a cell derived from a biological sample, such as a tissue sample or a body liquid sample of a subject, for example a blood sample.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

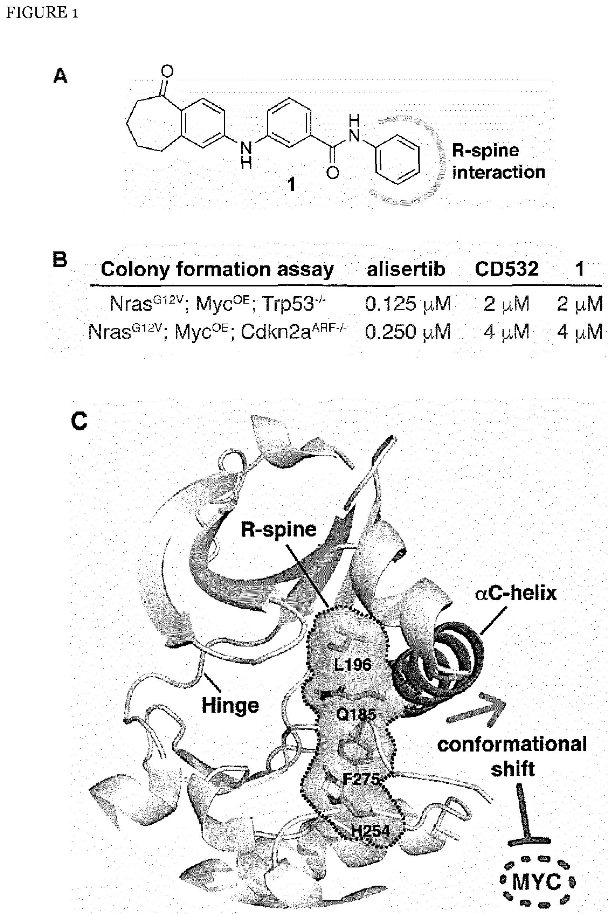

**Figure 1** **shows the initial hit compound and the structure of AURKA.** (A) Structure of initial hit compound. (B) Effective concentration of alisertib, CD532 and initial hit compound in a colony formation assay with NMP53^{-/-} (*Nras^{G12V}, MYC^{OE}, Trp53*^{*-*/*-*}; in the following referred to as "NMP53^{-/-}") and NMCdkn2a^{ARF-/-} (*Nras^{G12V}*, *MYC^{OE}, Cdkn2a*^{*ARF-*/*-*}; in the following referred to as "NMCdkn2a^{ARF-/-"}) cells. n=1. (C) AURKA structure in an active conformation (PDB ID: 6cpf). Visualization was done with the PyMOL Molecular Graphics System v. 2.0 (Schrödinger, LLC). R-spine (L196, Q185, F275, H254; highlighted by a dashed line) is located in close proximity to the αC-helix, which conformational shift (indicated with an arrow) interferes with MYC binding. The hinge region of AURKA is also indicated.

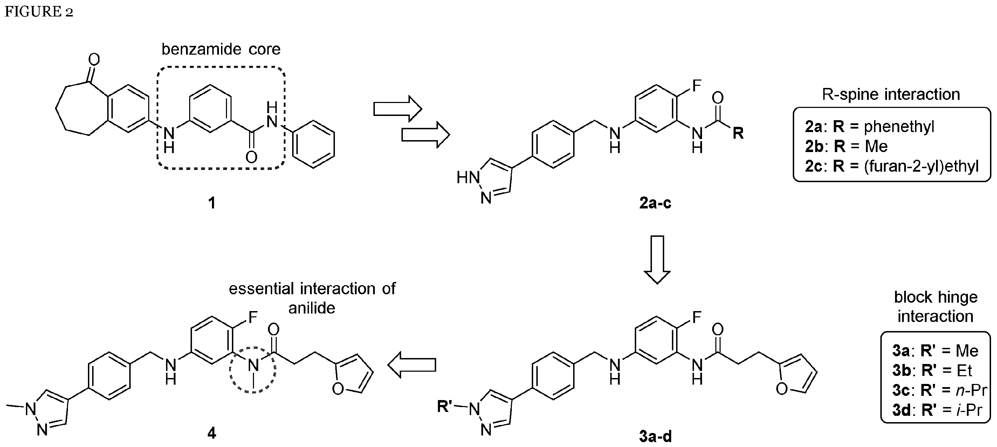

**Figure 2** **shows the development of compounds of this invention starting from the initial hit compound.** The 1H-pyrazole hinge binder was combined with a suitable terminal moiety R to result in compounds 2a [*N*-(5-((4-(1*H*-pyrazole-4-yl)benzyl)amino)-2-fluorophenyl)-3-phenylpropanamide], 2b [*N*-(5-((4-(1*H*-pyrazole-4-yl)benzyl)amino)-2-fluorophenyl)acetamide], and 2c [*N*-(5-((4-(1*H*-pyrazole-4-yl)benzyl)amino)-2-fluorophenyl)-3-(furan-2-yl)propanamide]. Blocking the hinge interaction R' eliminated the ATP-competitive character, leading to compounds 3a [*N*-(2-fluoro-5-((4-(1-methyl-1*H*-pyrazole-4-yl)benzyl)amino)phenyl)-3-(furan-2-yl)propanamide], 3b [*N*-(5-((4-(1-ethyl-1*H*-pyrazole-4-yl)benzyl)amino)-2-fluorophenyl)-3-(furan-2-yl)propanamide], 3c [*N*-(2-fluoro-5-((4-(1-propyl-1*H*-pyrazole-4-yl)benzyl)amino)phenyl)-3-(furan-2-yl)propanamide], 3d [*N*-(2-fluoro-5-((4-(1-isopropyl-1*H*-pyrazole-4-yl)benzyl)amino)phenyl)-3-(furan-2-yl)propanamide], and 4 [*N*-(2-fluoro-5-((4-(1-methyl-1*H*-pyrazole-4-yl)benzyl)amino)phenyl)-3-(furan-2-yl)-*N*-methylpro-panamide]. The amide was identified as essential moiety, as highlighted in compound 4.

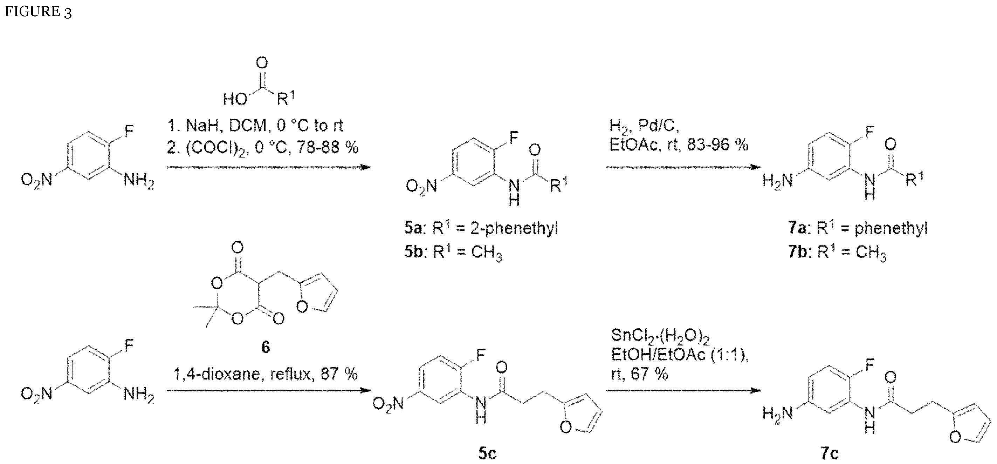

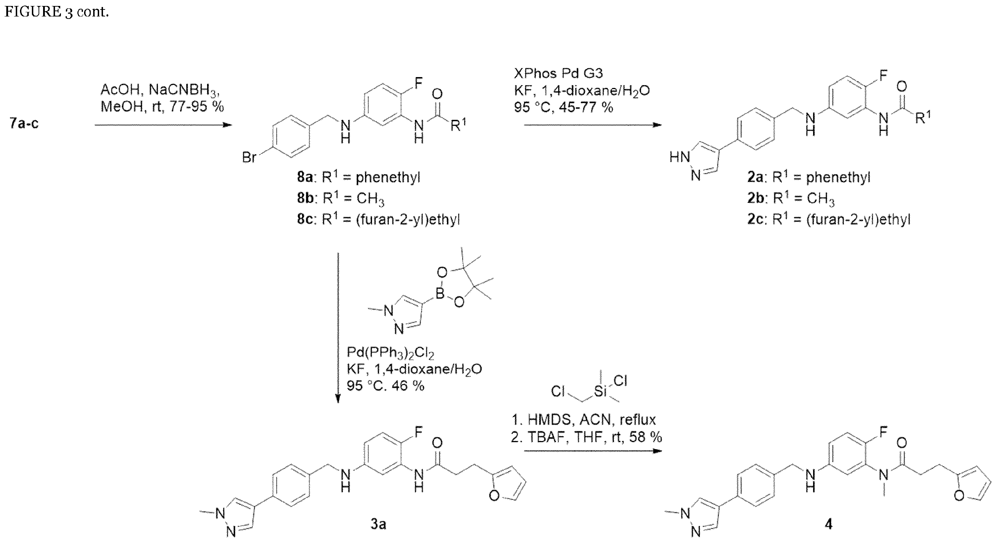

**Figure 3** **shows the synthesis of compounds 2a, 2b, 2c, 3a, and 4 of this invention.** Synthesis of compounds 2a [*N*-(5-((4-(1*H*-pyrazole-4-yl)benzyl)amino)-2-fluorophenyl)-3-phenylpropanamide], 2b [*N*-(5-((4-(1*H*-pyrazole-4-yl)benzyl)amino)-2-fluorophenyl)acetamide], 2c [*N*-(5-((4-(1*H*-pyrazole-4-yl)benzyl)amino)-2-fluorophenyl)-3-(furan-2-yl)propanamide], 3a [*N*-(2-fluoro-5-((4-(1-methyl-1*H*-pyrazole-4-yl)benzyl)amino)phenyl)-3-(furan-2-yl)propanamide] and 4 [*N*-(2-fluoro-5-((4-(1-methyl-1*H-*pyrazole-4-yl)benzyl)amino)phenyl)-3-(furan-2-yl)-*N*-methylpropanamide] is shown, starting from 2-fluoro-5-nitroaniline as core fragment via compounds 5a [*N*-(2-fluoro-5-nitrophenyl)-3-phenylpropanamide], 5b [N-(2-fluoro-5-nitrophenyl)acetamide], 5c [*N*-(2-fluoro-5-nitrophenyl)-3-(furan-2-yl)propanamide], 7a [*N*-(5-amino-2-fluorphenyl)-3-phenylpropanamid], 7b [N-(5-amino-2-fluorophenyl)acetamide], 7c [*N*-(5-amino-2-fluorophenyl)-3-(furan-2-yl)propanamide], 8a [*N*-(5-((4-bromobenzyl)amino)-2-fluorophenyl)-3-phenylpropanamide], 8b [*N*-(5-((4-bromobenzyl)amino)-2-fluorophenyl)acetamide], and 8c [*N*-(5-((4-bromobenzyl)amino)-2-fluorophenyl)-3-(furan-2-yl)propanamide]. DCM = dichloromethane, HMDS = 1,1,1,3,3,3-hexamethyldisilazane, ACN = acetonitrile, TBAF = tetrabutylammonium fluoride.

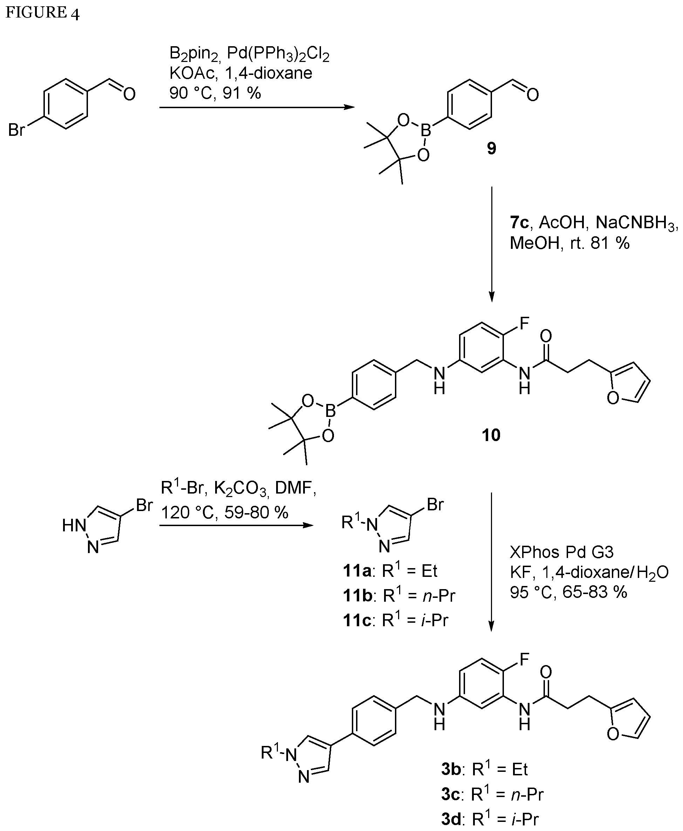

**Figure 4** **shows the synthesis of compounds 3b, 3c, and 3d of this invention.** Compounds 3b [*N*-(5-((4-(1-ethyl-1*H*-pyrazole-4-yl)benzyl)amino)-2-fluorophenyl)-3-(furan-2-yl)propanamide], 3c [*N*-(2-fluoro-5-((4-(1-propyl-1*H*-pyrazole-4-yl)benzyl)amino)phenyl)-3-(furan-2-yl)propanamide], and 3d [*N*-(2-fluoro-5-((4-(1-isopropyl-1*H*-pyrazole-4-yl)benzyl)amino)phenyl)-3-(furan-2-yl)propanamide] were synthesized via compound 9 [4-(4,4,5,5-Tetramethyl-1,3,2-dioxaborolane-2-yl)benzaldehyde] and compound 10 [*N*-(2-Fluoro-5-((4-(4,4,5,5-tetramethyl-1,3,2-dioxaborolane-2-yl)benzyl)amino)phenyl)-3-(furan-2-yl)propanamide]. Abbreviations: B₂pin₂ = 4,4,4',4',5,5,5',5'-octamethyl-2,2'-bi-1,3,2-dioxaborolane, DMF = N,N-dimethylformamide.

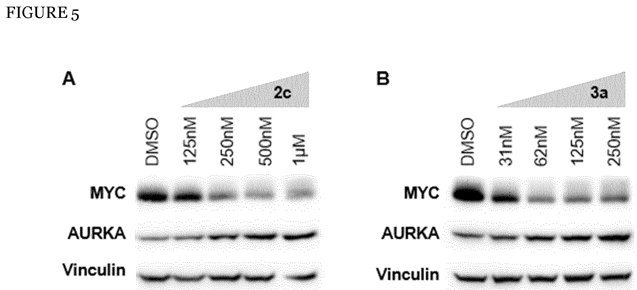

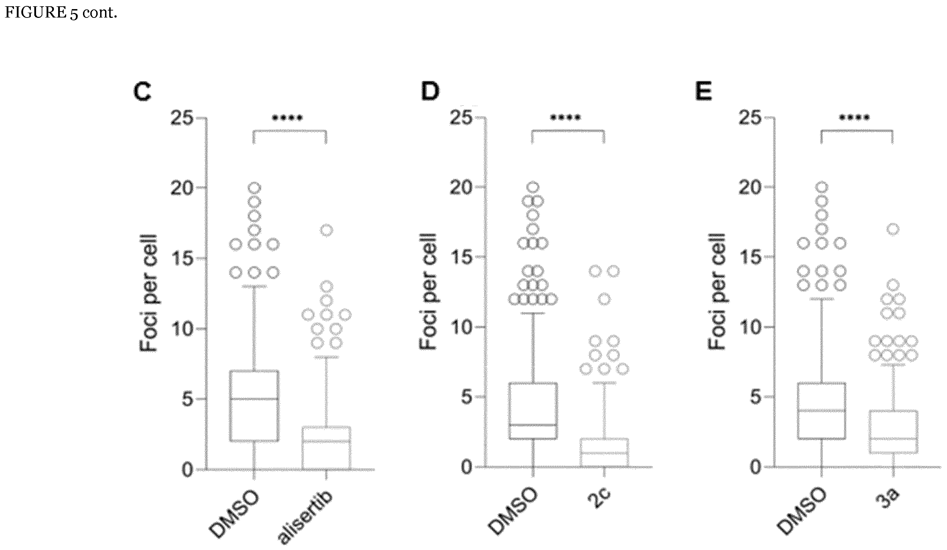

**Figure 5** **shows the effect of compound 2c and compound 3a on MYC and AURKA protein levels, as well as the interaction of MYC and AURKA in tumor cells.** A. Western blot analysis of MYC and AURKA in tumor cells (NMP53^{-/-} cells) 8 h after treatment with increasing concentrations of compound 2c [*N*-(5-((4-(1*H*-pyrazole-4-yl)benzyl)amino)-2-fluorophenyl)-3-(furan-2-yl)propanamide]. Vinculin served as loading control (n = 2) B. Western blot analysis of MYC and AURKA in tumor cells (NMP53^{-/-} cells) 8 h after treatment with increasing concentrations of compound 3a [*N*-(2-fluoro-5-((4-(1-methyl-1*H*-pyrazole-4-yl)benzyl)amino)phenyl)-3-(furan-2-yl)propanamide]. Vinculin served as loading control (n = 2). C-E show the summary of 2 (alisertib) or 3 (compound 2c, compound 3a) independent PLA experiments for MYC and AURKA in tumor cells (NMP53^{-/-} cells) 2 h after treatment with compound 2c and compound 3a and 24 h after treatment with alisertib. **** P < 0.0001, nonparametric Mann Whitney test.

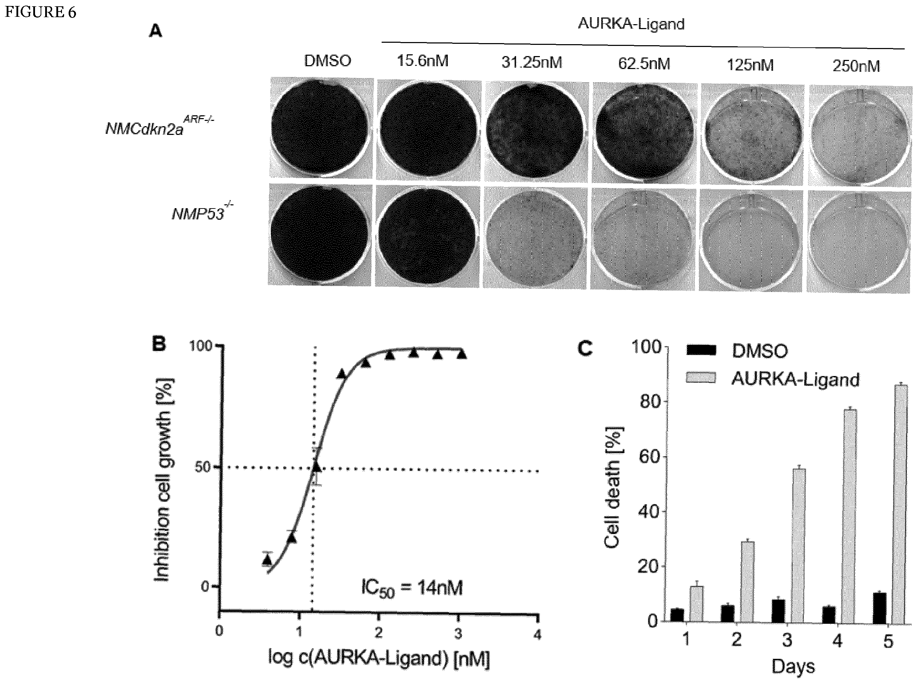

**Figure 6** **shows that the compounds of this invention act on malignant cells in the low nanomolar range and induce cell death.** (A) Colony formation assay of a tumor cell line (NMP53^{-/-} cells), incubated for 5 days with compound 3a [N-(2-fluoro-5-((4-(1-methyl-1H-pyrazole-4-yl)benzyl)amino)phenyl)-3-(furan-2-yl)-propanamide] as indicated, fixed and stained with crystal violet. (B) Viability measurement (XTT assay) of a tumor cell line (NMP53^{-/-}cells) treated for 3 days with a series of concentrations of compound 3a according to this invention. The determined IC50 of compound 3a is 14 nM. (C) Determination of the percentage of cell death (by means of propidium iodide staining) of a tumor cell line (NMP53^{-/-} cells) treated with compound 3a for 5 days.

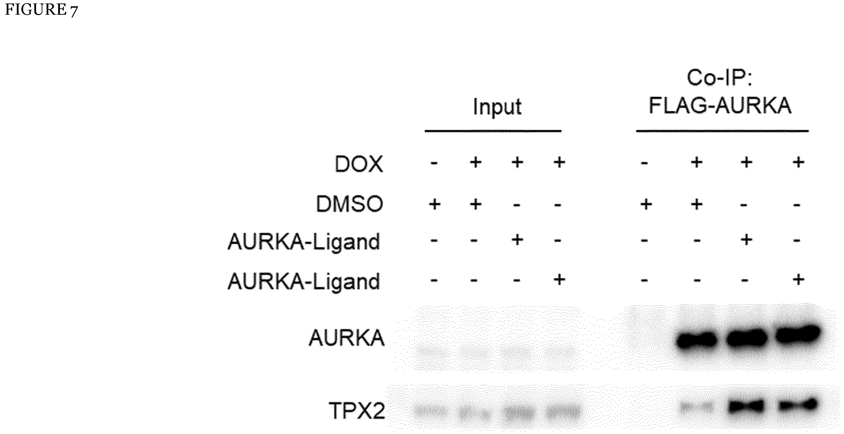

**Figure 7** **shows that the compounds of this invention induce an increased interaction of AURKA with TPX2.** Tumor cells (NMP53^{-/-} cells) expressing a doxycycline-inducible FLAG-AURKA construct were kept on doxycycline for 24 hours, synchronized with 50 ng/ml nocodazole for 6 hours and treated with two of the developed compounds or DMSO as a control. In particular, the top "AURKA-Ligand" refers to compound 3a [N-(2-fluoro-5-((4-(1-methyl-1H-pyrazole-4-yl)benzyl)amino)phenyl)-3-(furan-2-yl)¬propanamide], and the bottom "AURKA-Ligand" refers to compound 2c [N-(5-((4-(1H-pyrazole-4-yl)benzyl)amino)-2-fluorophenyl)-3-(furan-2-yl)propanamide]. Co-immunoprecipitations (Co-IP) were carried out with an antibody against FLAG. A DMSO-treated sample without FLAG-AURKA induction served as a control. The AURKA and TPX2 levels of the Co-IP samples and their input were determined by western blotting.
**Figure 8** **shows in vitro data on the induction of multipolar and monopolar spindles after application of the AURKA compounds of this invention.** The AURKA compounds of this invention trigger the formation of abnormal (multipolar and monopolar) spindles a few minutes after application. A. Representative images of immunofluorescence stainings with antibodies against α-tubulin (light grey) and DAPI (surrounded by a dashed white line) of a tumor cell line (NMP53^{-/-} cells) treated for 24 hours with compound 3a [N-(2-fluoro-5-((4-(1-methyl-1H-pyrazole-4-yl)benzyl)amino)phenyl)-3-(furan-2-yl)-propanamide] or DMSO as a control. B. Quantification of normal and abnormal spindles of immunofluorescence staining with antibodies against α-tubulin and DAPI of a tumor cell line (NMP53^{-/-} cells) after different treatment times with compound 3a according to this invention or DMSO as a control.

The sequences show: SEQ ID NO. 1 shows the sequence of human AURKA.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

### Abbreviations:

ACN: acetonitrile
ATP: adenosintriphosphate
AURKA: Aurora Kinase A
B₂pin₂: 4,4,4',4',5,5,5',5'-octamethyl-2,2'-bi-1,3,2-dioxaborolane
bHLH: basic helix-loop-helix (bHLH)
DCE: dichloroethane
DME: dimethyl ether
DCM: dichloromethane
DMF: N,N-dimethylformamide
EC: effective concentration
DMSO: dimethylsulfoxide
HCC: Hepatocellular carcinoma
HEPES: 2-(4-(2-hydroxyethyl)-1-piperazinyl)-ethansulfonsaure
HMDS: 1,1,1,3,3,3-hexamethyldisilazane
HPLC: high performance liquid chromatography
HPLC-ESI-TOF: HPLC coupled to electrospray ionization-time of flight-mass spectrometry
IC50: half maximal inhibitory concentration
IMAC: Immobilized Metal Affinity Chromatography
IPTG: Isopropyl β-d-1-thiogalactopyranoside
MAX: MYC-associated factor X
Me: methyl
MeOH: methanol
NaCl: sodium chloride
PPI: protein-protein interaction
R-spine: regulatory spine
RT: room temperature
TBAF: tetrabutylammonium fluoride
TCEP: tris(2-carboxyethyl)phosphin
THF: tetrabutylammoniumfluoride
TLC: thin layer chromatography
TPX2: targeting protein for Xklp2

The examples show:

### Example 1: Development of compounds for selectively targeting the protein-protein interaction of AURKA with binding proteins

The inventors developed compounds for selectively targeting the protein-protein interaction of AURKA with MYC and/or TPX2. *In silico* predictions of the MYC-AURKA interaction interface suggested that the MYC-AURKA interaction is located next to the αC-helix, at which AURKAs R-spine is anchored. Therefore, the inventors reasoned that addressing the R-spine and the adjacent area with a small molecule could induce the desired conformational shift preventing MYC interaction. Accordingly, unlike the previously disclosed compounds alisertib or CD532, the inventors focused their development of novel compounds on those compounds that target the regulatory spine of AURKA (R-spine, Figure 1 C).

To evaluate the effect of small molecules equipped with R-spine interacting moieties, the inventors screened a focused library of proprietary p38α MAP-Kinase inhibitors which were equipped with R-spine interacting pharmacophores. To this end, the inventors utilized a screening system as developed in Dauch *et al.*² The screening system is based on colony formation assays of cell lines isolated from *Nras*-driven, *Trp53*-deficient murine HCCs, whose development is dependent on MYC (NMP53^{-/-} cells), as well as *Nras*-driven, *Cdkn2a^{ARF}*-deficient murine HCCs, which develop independently of MYC (NMCdkn2a^{ARF-/-} cells). Importantly, NMP53^{-/-} cells are sensitive to AURKA inhibitors, which induce a conformational shift in AURKA, whereas NMCdkn2a^{ARF-/-} are not sensitive to such compounds.

For Colony Formation Assays, cells were seeded at a density of 50 000 cells per 6well and next day treated with a 2-fold dilution series of the indicated compounds or the highest corresponding DMSO concentration as control. After 5 days, the cells were fixed with a 10 % formaldehyde solution for 30 min, washed once with water, stained with a 0.5 % crystal violet solution for 30 min and washed twice with water. The compounds effective concentration (EC) was determined according to the lowest concentration in the dilution series, that inhibited cell growth substantially. The resulting benzamide containing hit structure 1, with a twofold selectivity towards NMP53^{-/-} cells (effective concentration on NMP53^{-/-} cells (EC): 2 µM, EC on Cdkn2a^{ARF-/-}: 4µM, Figure 1), served as the inventors' starting point for further compound development.

The inventors aimed at adapting the R-spine interacting scaffold and extending it with a suitable linker and a hinge binding moiety to gain enhanced binding affinity to AURKA. With a minimalist hinge binder and changing the benzamide to an anilide core, compound 2a [*N*-(5-((4-(1*H*-pyrazole-4-yl)benzyl)amino)-2-fluorophenyl)-3-phenylpropanamide] (Figure 2) was predicted to address the hinge region and additionally enable the R-spine interacting moiety to reach towards the αC-helix. This compound showed a clearly improved activity (EC on NMP53^{-/-}: 500 nM, Table 1) and a 4-fold increased selectivity for the NMP53^{-/-} cells.

By altering the terminal phenyl residue of compound 2a, the cellular efficacy of the developed compounds was further increased. The furane derivative (compound 2c [*N*-(5-((4-(1*H-*pyrazole-4-yl)benzyl)amino)-2-fluorophenyl)-3-(furan-2-yl)propanamide]) showed an up to 16-fold higher cellular activity (EC on NMP53^{-/-} cells: 125 nM, Table 1) compared to the initial hit 1 and a 4-fold increased efficacy compared to compound 2a [*N*-(5-((4-(1*H*-pyrazole-4-yl)benzyl)amino)-2-fluorophenyl)-3-phenylpropanamide]. Simultaneously, the selectivity was improved (up to 8-fold). In compound 2b [*N*-(5-((4-(1*H*-pyrazole-4-yl)benzyl)amino)-2-fluorophenyl)acetamide], the terminal residue was removed, which led to a complete loss of cellular activity (EC on NMP53^{-/} cells: >4.000 nM, Table 1), which outlines the importance of this moiety.

Due to the hinge interaction, compounds 2a [*N*-(5-((4-(1*H*-pyrazole-4-yl)benzyl)amino)-2-fluorophenyl)-3-phenylpropanamide], 2b [*N*-(5-((4-(1*H*-pyrazole-4-yl)benzyl)amino)-2-fluorophenyl)acetamide], and 2c [*N*-(5-((4-(1*H*-pyrazole-4-yl)benzyl)amino)-2-fluorophenyl)-3-(furan-2-yl)propanamide] still preserve an ATP-competitive character. Since the kinase activity is not related to the MYC/TPX2 -AURKA interaction and a strong inhibition of AURKA can lead to dose limiting toxicity, the inventors next weakened the interaction to the hinge region by blocking the 1*H*-pyrazole, which led to development of compounds 3a-d. When the inventors blocked the 1*H*-pyrazole of compounds 2a-c to weaken the interaction to the hinge region, the inventors expected a decreased binding affinity to AURKA and a reduced cellular activity due to the removal of a site of interaction from the (presumed) hinge binding moiety in AURKA. Interestingly, however, the inventors observed that 1-methyl-1*H*-pyrazol, as in the compound 3a [*N*-(2-fluoro-5-((4-(1-methyl-1*H*-pyrazole-4-yl)benzyl)amino)phenyl)-3-(furan-2-yl)-propanamide] (Figure 2) appeared even more potent in the screening assay, while displaying a similar selectivity (EC on NMP53^{-/-} cells: 31 nM, Table 1, see figure 6a). Sterically more demanding groups, as in compounds 3b [*N*-(5-((4-(1-ethyl-1*H*-pyrazole-4-yl)benzyl)amino)-2-fluorophenyl)-3-(furan-2-yl)propanamide], 3c [*N*-(2-fluoro-5-((4-(1-propyl-1*H*-pyrazole-4-yl)benzyl)amino)phenyl)-3-(furan-2-yl)propanamide], and 3d [*N*-(2-fluoro-5-((4-(1-isopropyl-1*H*-pyrazole-4-yl)benzyl)amino)phenyl)-3-(furan-2-yl)propanamide] led to lower cellular efficacy and loss of selectivity (EC on NMP53^{-/-} cells: 125 - >4000 nM, Table 1), indicating that methylation is the most promising option for eliminating hinge interaction while maintaining potency and selectivity, as in compound 3a. The inventors further evaluated the potential binding mode of compound 3a [*N*-(2-fluoro-5-((4-(1-methyl-1*H*-pyrazole-4-yl)benzyl)amino)phenyl)-3-(furan-2-yl)propanamide] by *in silico* docking, which suggested that the 1-methyl-1*H*-pyrazol could occupy the hydrophobic region I. Since the hinge interaction is abolished, the inventors reasoned that the main interaction to AURKA occurs at the anilide core. The importance of this structural feature is demonstrated by compound 4 (Figure 2). By blocking the hydrogen donor feature of the amide moiety, the cellular potency was dramatically decreased (EC on NMP53^{-/-}cells: >4000 nM, Table 1).

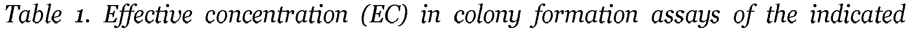

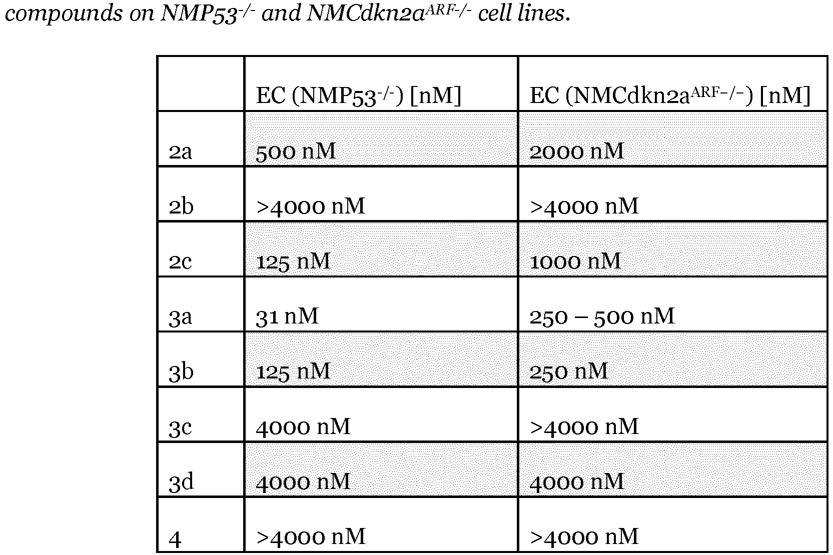

Figure 6 shows that the AURKA compounds of this invention act on malignant cells in the low nanomolar range and induce cell death. Importantly, the amide and a suitable terminal moiety reaching towards the αC-helix are essential for the activity of the compounds of this invention. Furthermore, the combination with benzyl linked 1*H*-pyrazole additionally increases the cellular potency and ensures a striking selectivity for the NMP53^{-/-} cells (as in compound 2c [*N*-(5-((4-(1*H*-pyrazole-4-yl)benzyl)amino)-2-fluorophenyl)-3-(furan-2-yl)propanamide]). Finally, methylation of the 1*H*-pyrazole uncouples cellular potency and AURKA inhibition (compound 3a [*N*-(2-fluoro-5-((4-(1-methyl-1*H*-pyrazole-4-yl)benzyl)amino)phenyl)-3-(furan-2-yl)propanamide]).

### Example 2: Preparation of the compounds of this invention

The compounds were synthesized starting from 2-fluoro-5-nitroaniline as core fragment (Figure 3). 2-Fluoro-5-nitroaniline (1.00 eq.) was added portion wise to an ice cold, argon flushed solution of sodium hydride (1.05 eq., 60 % w/w in mineral oil) in dry DCM (4 mL/mmol). After the initial exothermic reaction, the solution was warmed to RT. In a second vessel, oxalyl chloride (1.05 eq.) was added to an ice cold solution of hydrocinnamic acid or acetic acid (1.05 eq.) in dry DCM (4 mL/mmol) followed by the addition of a catalytic amount of dry DMF. After complete activation of the carboxylic acid, this solution was slowly added to the first vessel with a dropping funnel. After complete reaction (as determined by TLC), brine was added slowly at 0°C and the layers were separated. The aqueous layer was extracted two more times with DCM. The combined organic layers were washed with brine, dried over Na₂SO₄ and the solvent was removed *in vacuo.* The residue was purified via flash chromatography.

A subsequent amide synthesis with in *situ* generated hydrocinnamic acid chloride or acetyl chloride yielded compound 5a [*N*-(2-fluoro-5-nitrophenyl)-3-phenylpropanamide] and compound 5b [N-(2-fluoro-5-nitrophenyl)acetamide] (Figure 3). Compound 5c [*N*-(2-fluoro-5-nitrophenyl)-3-(furan-2-yl)propanamide] was obtained via a modified, high yielding *N-*acetylation method published by Li *et al.*¹ A dry, argon flushed reaction vessel was charged with 2-fluoro-5-nitroaniline and compound 6 [5-(2-furanylmethyl)-2,2-dimethyl-1,3-Dioxane-4,6-dione]. Dry 1,4-dioxane (4 mL/mmol) was added and the solution was refluxed for 6 h or overnight and cooled to RT. After evaporation of the solvent, the residue was diluted in ethyl acetate and washed with NaHCO_{3(aq., sat.)}, water and brine. The organic layer was dried over Na₂SO₄ and the solvent was removed *in vacuo.* The obtained solid was washed with diethyl ether/n-hexane (9:1) to yield compound 5c [*N*-(2-fluoro-5-nitrophenyl)-3-(furan-2-yl)propanamide].

Pd/C (0.10 eq., 10 % Pd basis) was added to a stirred solution of the nitroaniline compounds 5a [*N*-(2-fluoro-5-nitrophenyl)-3-phenylpropanamide] or 5b [*N*-(2-fluoro-5-nitrophenyl)acetamide) in ethyl acetate/methanol (95:5, 10 mL/mmol) at RT (Figure 3). A stream of H₂ was applied until complete reduction of the nitro group (TLC). The catalyst was filtered, the filtrate was washed 2 times with water and the organic layer was dried over Na₂SO₄. After evaporation of the solvent, the aniline compounds 7a [*N*-(5-amino-2-fluorphenyl)-3-phenylpropanamid] and 7b [N-(5-amino-2-fluorophenyl)acetamide] were obtained as white solids (Figure 3).

To synthesize compound 7c [*N*-(5-amino-2-fluorophenyl)-3-(furan-2-yl)propanamide], compound 5c [*N*-(2-fluoro-5-nitrophenyl)-3-(furan-2-yl)propanamide] (1.2 g, 4.31 mmol, 1.00 eq.) was solved in ethyl acetate and ethanol (1:1, 3 mL/mmol) and SnCl₂(H₂O)₂ (2.92 g, 12.94 mmol, 3.00 eq.) was added in one portion (Figure 3). The mixture was stirred at reflux for 5 h and cooled to RT. The excess tin chloride was precipitated with NaHCO_{3(aq., sat.)} and filtered off. The layers were separated and the aqueous layer was extracted two times with ethyl acetate. The combined organic layers were dried over Na₂SO₄ and the solvent was removed *in vacuo.* The residue was purified via flash chromatography to yield compound 7c [*N*-(5-amino-2-fluorophenyl)-3-(furan-2-yl)propanamide] as white solid (Figure 3).

After reduction of compounds 7a [*N*-(5-amino-2-fluorphenyl)-3-phenylpropanamid], 7b [N-(5-amino-2-fluorophenyl)acetamide], and/or 7c [*N*-(5-amino-2-fluorophenyl)-3-(furan-2-yl)propanamide], a reductive amination with 4-bromobenzaldehyde yielded the compounds 8a [*N*-(5-((4-bromobenzyl)amino)-2-fluorophenyl)-3-phenylpropanamide], 8b [*N*-(5-((4-bromobenzyl)amino)-2-fluorophenyl)acetamide], and 8c [*N*-(5-((4-bromobenzyl)amino)-2-fluorophenyl)-3-(furan-2-yl)propanamide] (Figure 3).

Compounds 8a [*N*-(5-((4-bromobenzyl)amino)-2-fluorophenyl)-3-phenylpropanamide], 8b [*N*-(5-((4-bromobenzyl)amino)-2-fluorophenyl)acetamide], and 8c [*N*-(5-((4-bromobenzyl)amino)-2-fluorophenyl)-3-(furan-2-yl)propanamide] (Figure 3) were synthesized by reduction of compounds 7a, 7b, and/or 7c. 4-Bromobenzaldehyde (1.00 eq.) was solved in methanol and a catalytic amount of acetic acid was added. After stirring for 15 min at RT, compounds 7a [*N*-(5-amino-2-fluorphenyl)-3-phenylpropanamid], 7b [*N*-(5-amino-2-fluorophenyl)acetamide], and/or 7c [*N*-(5-amino-2-fluorophenyl)-3-(furan-2-yl)propanamide] (1.00 eq.) was added and the mixture was stirred for further 30-60 min. Upon precipitation of a white solid, sodium cyanoborohydride (1.50 eq.) was added portion wise. After completion (as determined by TLC) the solvent was evaporated and the residue was diluted with DCM and water. The layers were separated and the aqueous layer was two times extracted with DCM. The combined organic layers were dried over Na₂SO₄ and the solvent was removed *in vacuo* and purified via flash-chromatography, yielding compounds 8a [*N*-(5-((4-bromobenzyl)amino)-2-fluorophenyl)-3-phenylpropanamide], 8b [*N*-(5-((4-bromobenzyl)amino)-2-fluorophenyl)acetamide], and 8c [*N*-(5-((4-bromobenzyl)amino)-2-fluorophenyl)-3-(furan-2-yl)propanamide] (Figure 3).

Synthesis of the 1*H*-pyrazole compounds 2a-c was performed by Suzuki coupling with (1*H*-pyrazol-4-yl)boronic acid (Figure 3). An argon flushed reaction vessel was charged with a corresponding aryl halide (compound 8a [*N*-(5-((4-bromobenzyl)amino)-2-fluorophenyl)-3-phenylpropanamide], 8b [*N*-(5-((4-bromobenzyl)amino)-2-fluorophenyl)acetamide], and/or 8c [*N*-(5-((4-bromobenzyl)amino)-2-fluorophenyl)-3-(furan-2-yl)propanamide], 1.00 eq.), XPhos Pd G3 (0.05 eq.), 1H-pyrazole-4-boronic acid (1.5 eq.) and potassium fluoride (3.00 eq.). A mixture of 1,4-dioxane and water (4:1) was flushed with argon for 10 min and added to the reaction vessel (5 mL/mmol). The reaction was heated to 95 °C and monitored via TLC. Upon completion, the reaction was cooled to RT and water/ethyl acetate (1:1) was added. The layers were separated and the aqueous layer was extracted with ethyl acetate two more times. The combined organic layers were dried over Na₂SO₄ and the solvent was evaporated. The raw material was purified via flash-chromatography to yield compounds 2a [*N*-(5-((4-(1*H*-pyrazole-4-yl)benzyl)amino)-2-fluorophenyl)-3-phenylpropanamide], 2b [*N*-(5-((4-(1H-pyrazole-4-yl)benzyl)amino)-2-fluorophenyl)acetamide], and 2c [*N*-(5-((4-(1*H*-pyrazole-4-yl)benzyl)amino)-2-fluorophenyl)-3-(furan-2-yl)propanamide] (Figure 3).

Compound 3a [*N*-(2-fluoro-5-((4-(1-methyl-1*H*-pyrazole-4-yl)benzyl)amino)phenyl)-3-(furan-2-yl)propanamide] was obtained starting from compound 8c [*N*-(5-((4-bromobenzyl)amino)-2-fluorophenyl)-3-(furan-2-yl)propanamide] (Figure 3). An argon flushed reaction vessel was charged with compound 8c [*N*-(5-((4-bromobenzyl)amino)-2-fluorophenyl)-3-(furan-2-yl)propanamide] (100 mg, 0.24 mmol, 1.00 eq.), Pd(PPh₃)₂Cl₂ (8 mg, 0.01 mmol, 0.05 eq.), 1-methyl-4-(4,4,5,5-tetramethyl-1,3,2-dioxaborolane-2-yl)-1*H*-pyrazole (60 mg, 0.29 mmol, 1.2 eq.) and potassium fluoride (28 mg, 0.48 mmol, 2.00 eq.). A mixture of 1,4-dioxane and water (4:1) was flushed with argon for 10 min and added to the reaction vessel (5 mL/mmol). The reaction was heated to 95 °C and monitored via TLC. Upon completion, the reaction was cooled to RT and water/ethyl acetate (1:1) was added. The layers were separated and the aqueous layer was extracted with ethyl acetate two more times. The combined organic layers were dried over Na₂SO₄ and the solvent was evaporated. Purification via flash-chromatography yielded compound 3a [*N*-(2-fluoro-5-((4-(1-methyl-1*H*-pyrazole-4-yl)benzyl)amino)phenyl)-3-(furan-2-yl)propanamide] as white solid (Figure 3).

Compound 4 [*N*-(2-fluoro-5-((4-(1-methyl-1*H*-pyrazole-4-yl)benzyl)amino)phenyl)-3-(furan-2-yl)-*N*-methylpropanamide] was obtained by selective methylation of the amide moiety, facilitated by a Chapman type rearrangement with chloro(chloromethyl)dimethylsilane, starting directly from compound 3a [*N*-(2-fluoro-5-((4-(1-methyl-1*H*-pyrazole-4-yl)benzyl)amino)phenyl)-3-(furan-2-yl)propanamide] (Figure 3). 1,1,1,3,3,3-Hexamethylsilazane (19 mg, 0.12 mmol, 1.00 eq.) was added to a solution of compound 3a [*N*-(2-fluoro-5-((4-(1-methyl-1*H*-pyrazole-4-yl)benzyl)amino)phenyl)-3-(furan-2-yl)propanamide] (50 mg, 0.12 mmol, 1.00 eq.) in dry acetonitrile and stirred for 5 h at 50 °C. Then, chloro-(chloromethyl)-dimethylsilane (32 µL, 0.24 mmol, 2.00 eq.) was added and the reaction was refluxed overnight. After cooling to RT, the solvent was evaporated and the obtained residue was diluted with THF. Tetrabutylammoniumfluoride (1M in THF, 4.00 eq.) was added and stirred until the reaction was completed (TLC/TLC-MS). The solvent was removed *in vacuo* and ethyl acetate/water was added, the layers were separated and the aqueous layer was extracted with ethyl acetate one more time. The combined organic layers were dried over Na₂SO₄, the solvent was evaporated and the obtained solid was purified via flash-chromatography to yield compound 4 [*N*-(2-fluoro-5-((4-(1-methyl-1*H*-pyrazole-4-yl)benzyl)amino)phenyl)-3-(furan-2-yl)-*N*-methylpropanamide] as a white solid (Figure 3).

For the synthesis of further *N*-substituted pyrazoles, the boronic acid pinacol ester compound 9 [4-(4,4,5,5-Tetramethyl-1,3,2-dioxaborolane-2-yl)benzaldehyde] was synthesized via Miyaura borylation starting from 4-bromobenzaldehyde (Figure 4). An argon flushed reaction vessel was charged with 4-bromobenzaldehyde (1.4 g, 7.57. mmol, 1.00 eq.), bis(pinacolato)diboron (2.02 g, 7.95 mmol, 1.05 eq.), Pd(PPh₃)₂Cl₂ (106 mg, 0.15 mmol, 0.02 eq.) and oven dried potassium acetate (1.49 g, 15.13 mmol, 2.00 eq.). The solids were solved in dry 1,4-dioxane (5 mL/mmol) and the solution was stirred at 90°C. After the reaction was completed (TLC), it was cooled to RT, filtrated and the solvent was evaporated. The residue was diluted with ethylacetate and washed with water and brine. The organic layer was dried over Na₂SO₄ and the solvent was removed *in vacuo.* The raw material was purified via flash-chromatography (100 % n-hexane to n-hexane/ethyl acetate 8:2 for 10 CV, gradient) and the resulting solid was washed with n-pentane to yield compound 9 [4-(4,4,5,5-Tetramethyl-1,3,2-dioxaborolane-2-yl)benzaldehyde] (Figure 4).

Compound 9 [4-(4,4,5,5-Tetramethyl-1,3,2-dioxaborolane-2-yl)benzaldehyde] (450 mg, 1.94 mmol, 1.00 eq.) was then solved in methanol and a catalytic amount of acetic acid was added. After stirring for 15 min at RT, compound 7c [*N*-(5-amino-2-fluorophenyl)-3-(furan-2-yl)propanamide] (481 mg, 1.94 mmol, 1.00 eq.) was added and the mixture was stirred for further 45 min. Upon precipitation of a white solid sodium cyanoborohydride (182 mg, 2.91 mmol, 1.50 eq.) was added portion wise. After completion (as determined by TLC), the solvent was evaporated and the residue was diluted with DCM and water. The layers were separated and the aqueous layer was two times extracted with DCM. The combined organic layers were dried over Na₂SO₄ and the solvent was removed *in vacuo* and purified via flash-chromatography (100 % n-hexane to n-hexane/ethyl acetate 7:3 for 10 CV, gradient) to yield compound 10 [N-(2-Fluoro-5-((4-(4,4,5,5-tetramethyl-1,3,2-dioxaborolane-2-yl)benzyl)amino)phenyl)-3-(furan-2-yl)propanamide] as white solid (Figure 4).

Compounds 11a [4-bromo-1-ethyl-1*H*-pyrazole], 11b [4-bromo-1-propyl-1*H*-pyrazole], and 11c [4-bromo-1-isopropyl-1*H*-pyrazole] were obtained by *N*-alkylation of 4-bromo-1*H-*pyrazole with the corresponding alkyl halides (Figure 4). Compound 11a was obtained by reaction with ethyl iodide, compound 11b was obtained by reaction with n-propyl iodide, and compound 11c was obtained by reaction with isopropyl iodide. 3-Brom-1*H*-pyrazole (1.00 eq.), the Bromoalkane (1.05 eq.) and K₂CO₃ (1.02 eq.) were applied to a screw cap reaction vessel and solved in dry DMF (4 mL/mmol). The reaction was stirred at 120 °C overnight. After cooling to RT, the solvent was reduced to ~40 % volume and brine was added. The layers were separated and the aqueous layer was extracted with DCM two times. The organic layers were dried over Na₂SO₄ and the solvent was removed *in vacuo* to yield the corresponding pyrazole halide compounds 11a [4-bromo-1-ethyl-1*H*-pyrazole], 11b [4-bromo-1-propyl-1*H*-pyrazole], and 11c [4-bromo-1-isopropyl-1*H*-pyrazole] (Figure 4).

The subsequent Suzuki coupling utilizing compound 10 [*N*-(2-Fluoro-5-((4-(4,4,5,5-tetramethyl-1,3,2-dioxaborolane-2-yl)benzyl)amino)phenyl)-3-(furan-2-yl)propanamide] yielded the *N*-alkylated pyrazole compounds 3b [*N*-(5-((4-(1-ethyl-1*H*-pyrazole-4-yl)benzyl)amino)-2-fluorophenyl)-3-(furan-2-yl)propanamide], 3c [*N*-(2-fluoro-5-((4-(1-propyl-1*H*-pyrazole-4-yl)benzyl)amino)phenyl)-3-(furan-2-yl)propanamide], and 3d [*N*-(2-fluoro-5-((4-(1-isopropyl-1*H*-pyrazole-4-yl)benzyl)amino)phenyl)-3-(furan-2-yl)propanamide] (Figure 4). An argon flushed reaction vessel was charged with the corresponding aryl halide (compounds 11a [4-bromo-1-ethyl-1*H*-pyrazole], 11b [4-bromo-1-propyl-1*H*-pyrazole], and 11c [4-bromo-1-isopropyl-1*H*-pyrazole], 1.00 eq.), XPhos Pd G3 (0.05 eq.), compound 10 [*N*-(2-Fluoro-5-((4-(4,4,5,5-tetramethyl-1,3,2-dioxaborolane-2-yl)benzyl)amino)phenyl)-3-(furan-2-yl)propanamide] (1.5 eq.) and potassium fluoride (2.00 eq.). A mixture of 1,4-dioxane and water (4:1) was flushed with argon for 10 min and added to the reaction vessel (5 mL/mmol). The reaction was heated to 95 °C and monitored via TLC. Upon completion, the reaction was cooled to RT and water/ethyl acetate (1:1) was added. The layers were separated and the aqueous layer was extracted with ethyl acetate two more times. The organic layers were dried over Na₂SO₄ and the solvent was evaporated. The raw material was purified via flash-chromatography to yield compounds 3b [*N*-(5-((4-(1-ethyl-1*H*-pyrazole-4-yl)benzyl)amino)-2-fluorophenyl)-3-(furan-2-yl)propanamide], 3c [*N*-(2-fluoro-5-((4-(1-propyl-1*H*-pyrazole-4-yl)benzyl)amino)phenyl)-3-(furan-2-yl)propanamide], and 3d [*N*-(2-fluoro-5-((4-(1-isopropyl-1*H*-pyrazole-4-yl)benzyl)amino)phenyl)-3-(furan-2-yl)propanamide] (Figure 4).

### Example 3: The compounds of this invention significantly reduce the MYC-AURKA interaction

As the compounds 2c [*N*-(5-((4-(1*H*-pyrazole-4-yl)benzyl)amino)-2-fluorophenyl)-3-(furan-2-yl)propanamide] and 3a [*N*-(2-fluoro-5-((4-(1-methyl-1*H*-pyrazole-4-yl)benzyl)amino)phenyl)-3-(furan-2-yl)propanamide] showed the most promising results in the inventors' primary screening assay, the inventors studied their effect on a cellular level more extensively. First, the inventors used Western Blot Analysis to determine the effect of compounds 2c [*N*-(5-((4-(1*H*-pyrazole-4-yl)benzyl)amino)-2-fluorophenyl)-3-(furan-2-yl)propanamide] and 3a [*N*-(2-fluoro-5-((4-(1-methyl-1*H*-pyrazole-4-yl)benzyl)amino)phenyl)-3-(furan-2-yl)-propanamide] on cellular MYC levels.

For western blot analysis, 2x10⁶ cells were seeded per 10 cm dish and next day treated with the indicated compound concentrations. Cells were collected at the mentioned time points and lysed in lysis buffer (20 mM Tris-HCl pH 7.5, 150 mM NaCl, 1 mM Na₂EDTA, 1 mM EGTA, 1 % Triton X-100, PhosSTOP EASYpack Phosphatase Inhibitor Cocktail Tablet (Roche), cOmplete Mini EDTA-free EASYpack Protease Inhibitor Cocktail Tablets (Roche) and 1/6 volume freshly added 8 M urea). After addition of 4x Laemmli buffer (200 mM Tris-HCl pH 6.8, 8 % SDS, 4 % β-mercaptoethanol, 40 % glycerol, 0.33 % bromophenol blue) the lysates were boiled for 5 min at 95 °C. For western blot analysis, proteins were separated by SDS-polyacrylamide electrophoresis (using 25 µg of each sample) and transferred onto PVDF Immobilon-P membranes (Millipore). The blots were incubated with antibodies specific to MYC (Abcam ab32072, 1:1 000), AURKA (Abcam ab13824, 1:1000) and Vinculin (Sigma Aldrich V9131-2mL 1:10 000), followed by peroxidase-conjugated secondary antibodies. Proteins were detected by ChemiDoc MP Imaging System (Bio-Rad) using Clarity Western ECL Substrate (Bio-Rad).

The inventors observed that after treatment with compound 2c [*N*-(5-((4-(1*H*-pyrazole-4-yl)benzyl)amino)-2-fluorophenyl)-3-(furan-2-yl)propanamide] and compound 3a [*N*-(2-fluoro-5-((4-(1-methyl-1*H*-pyrazole-4-yl)benzyl)amino)phenyl)-3-(furan-2-yl)propanamide], MYC levels were reduced in a concentration dependent manner in NMP53^{-/-} cells (Figure 5 A-B).

To examine whether this MYC degradation is associated with MYC-AURKA PPI disruption, the inventors performed proximity ligation assays (PLA) in NMP53^{-/-} cells. For Proximity Ligation Assay (PLA), 50 000 cells were seeded on 12 mm glass slides placed in 6well plates. On the next day, the cells were treated with the indicated compounds or the corresponding DMSO concentration for 2 h (compound 2c [*N*-(5-((4-(1*H*-pyrazole-4-yl)benzyl)amino)-2-fluorophenyl)-3-(furan-2-yl)propanamide] and compound 3a [*N*-(2-fluoro-5-((4-(1-methyl-1*H-*pyrazole-4-yl)benzyl)amino)phenyl)-3-(furan-2-yl)propanamide]) or 24 h (alisertib) and fixed for 10 min in 100 % MeOH at -20°C. The glass slides were removed from the 6well plates and the cells permeabilized with TBS + 0.3 % Triton X-100 for 5 min. After washing with PBS and blocking with 5 % BSA in TBS + 0.05 % Tween for 30 min, the slides were incubated with MYC (Abcam ab32072, 1:200) and AURKA (Abcam ab13824, 1:200) specific antibodies in 1 % BSA in TBS + 0.05 % Tween for 120 min at RT and washed 3 times with PBS for 5 min, wherein the MYC/TPX2 and AURKA specific antibodies are derived from a different species. After a 20 min preincubation of two PLA probes, directed against either species of the MYC/TPX2 and AURKA specific antibodies, at RT (DUO92001 and DUO92005 Sigma Aldrich, diluted 1:10 in 1 % BSA in TBS + 0.05 % Tween), the slides were incubated with the probes for 60 min at 37 °C in a humidified chamber. The slides were washed 3 times with 1x wash buffer A (Sigma Aldrich DUO82049) for 5 min and incubated with the ligase solution (ligase diluted 1:40 in 5x ligase buffer and ddH₂O, Sigma Aldrich DUO92008) for 60 min at 37 °C in a humidified chamber. Of note, upon washing the slides with the ligase solution, probes directed against either species of the MYC/TPX2 and AURKA specific antibodies are ligated, being indicative for a short distance and/or a binding between MYC/TPX2 and AURKA. After washing the slides 2 times for 5 min, the slides were incubated with polymerase solution (polymerase diluted 1:80 in 5x polymerase buffer and ddH₂O, Sigma Aldrich DUO92008) light protected for 120 min at 37 °C in a humidified chamber to amplify the ligated probes. The slides were washed 2 times in 1x Wash Buffer B (Sigma Aldrich DUO82049) for 10 min and once in 0.01x wash Buffer B for 1 min and mounted on a cover slip using Duolink *In Situ* Mounting Medium with DAPI (Sigma Aldrich DUO82040). Nail polish was used to seal the edges. The PLA foci (i.e. ligated probes) were visualized with an Olympus BX63 microscope equipped with an Olympus DP80 camera at 60-fold magnification. Foci are indicative for a close proximity between MYC/TPX2 and AURKA, induced by, e.g. an interaction between MYC/TPX2 and AURKA. The foci per cell were counted in a way that the cytoplasmic foci are counted to the cell with the closest nucleus (DAPI). Significance was calculated with the GraphPad Prism 8.4.0 software (GraphPad Software, La Jolla, CA) by using the nonparametric Mann Whitney test.

Indeed, the inventors observed a significant reduction of the MYC-AURKA interaction upon treatment with compound 2c [*N*-(5-((4-(1*H*-pyrazole-4-yl)benzyl)amino)-2-fluorophenyl)-3-(furan-2-yl)propanamide] (66,7% reduction compared to DMSO; Figure 5 C) and compound 3a [*N*-(2-fluoro-5-((4-(1-methyl-1*H*-pyrazole-4-yl)benzyl)amino)phenyl)-3-(furan-2-yl)-propanamide] (50% reduction compared to DMSO; Figure 5 D), which was comparable to the effect of alisertib (60% reduction compared to DMSO; Figure 5 E). The PLA was performed 2 h after treatment with compound 2c [*N*-(5-((4-(1*H*-pyrazole-4-yl)benzyl)amino)-2-fluorophenyl)-3-(furan-2-yl)propanamide] and compound 3a [*N*-(2-fluoro-5-((4-(1-methyl-1*H*-pyrazole-4-yl)benzyl)amino)phenyl)-3-(furan-2-yl)propanamide] (24 h after treatment with alisertib) to ensure that the diminished interaction is based on a direct effect of compound 2c [*N*-(5-((4-(1*H-*pyrazole-4-yl)benzyl)amino)-2-fluorophenyl)-3-(furan-2-yl)propanamide] or compound 3a [*N-*(2-fluoro-5-((4-(1-methyl-1*H*-pyrazole-4-yl)benzyl)amino)phenyl)-3-(furan-2-yl)propanamide], and not a result of altered MYC expression levels.

### Example 4: Determining the cellular IC50 values and the effect of the compounds of this invention on the activity of AURKA

The cell viability assay (XTT-Assay) was used to determine cellular IC50 values. 1 000 cells were seeded per 96well and treated with a 2-fold dilution series of the compounds on the next day. As a control, the cells were treated with DMSO according to the highest compound concentration (negative control) or 1.5 mM chloroquine (positive control). 3 days after treatment with the respective compound, or DMSO/chloroquine as a control, the XTT Assay was conducted according to the manufacturer's instructions (Invitrogen, X6493). The absorbance was measured with the Infinite M Plex plate reader (Tecan) at 450 nm. The IC50 was calculated by variable slope nonlinear regression utilizing GraphPad Prism 8.4.0 software (GraphPad Software, La Jolla, CA), with the DMSO treated cells resembling 100 % cell viability and the chloroquine treated cells resembling 0 % cell viability.

To measure the effect of the compounds on the activity of AURKA, the IC50 of the compounds was determined with the ADP-Glo^{™} Kinase Assay (Promega, V9101) and the Aurora A Kinase Enzyme System (Promega, V1931). The assay was performed according to the manufacturer's instructions in a white 384-well plate format (Corning Assay Plate, LOT 32216024) by using 1.4 ng/µl Aurora A, 25 mM ATP and 0.1 µg/µl myelin basic protein (MBP) substrate in a total volume of 6 µl (transferred with Integra viaflo 96/384 handheld channel pipette). To generate a dose-response curve, a 3-fold serial dilution of the compounds was tested starting from 1 µM. The kinase was pre-incubated for 10 min at RT with the respective compound, before the substrate mix containing MBP and ATP was added. After 60 min at RT, 6µl of the ADP-Glo reagent were added and incubated for another 60 min at RT. Afterwards 12 µl of the detection reagent were added and incubated for 30 min at RT before the luminescence was measured with an integration time of 500 ms by using a Filter Max F5 multi-mode microplate reader from Molecular Devices. As a negative control, the reaction was performed with MBP, ATP and Aurora A only. The positive control was performed with inactive Aurora A (boiled for 10 min), MBP and ATP. Each reaction was carried out in quadruplicates and the IC50 was calculated by variable slope nonlinear regression utilizing GraphPad Prism 8.4.0 software (GraphPad Software, La Jolla, CA), with the negative control resembling 0 % kinase inhibition and the positive control resembling 100 % kinase inhibition.

By comparing the compounds cellular IC50 with their AURKA inhibition, compound 2c [*N*-(5-((4-(1*H*-pyrazole-4-yl)benzyl)amino)-2-fluorophenyl)-3-(furan-2-yl)propanamide] and compound 3a [*N*-(2-fluoro-5-((4-(1-methyl-1*H*-pyrazole-4-yl)benzyl)amino)phenyl)-3-(furan-2-yl)propanamide] displayed different properties. While compound 2c [*N*-(5-((4-(1*H*-pyrazole-4-yl)benzyl)amino)-2-fluorophenyl)-3-(furan-2-yl)propanamide] represents an amphosteric inhibitor - targeting the MYC-AURKA interaction as well as AURKAs catalytic activity - with a cellular IC50 of 60 +/- 8 nM (XTT, n=3) and an IC₅₀(AURKA) of 259 +/- 4,3 nM (ADP-Glo Assay, n=2), compound 3a [*N*-(2-fluoro-5-((4-(1-methyl-1*H*-pyrazole-4-yl)benzyl)amino)phenyl)-3-(furan-2-yl)propanamide], with a cellular IC₅₀ of 13 +/- 1 nM (XTT, n=3, see figure 6b), exhibited no kinase inhibition, acting as PPI inhibitor only.

Moreover, the comparison between cellular and kinase IC₅₀ also validated that compound 2c [*N-*(5-((4-(1*H*-pyrazole-4-yl)benzyl)amino)-2-fluorophenyl)-3-(furan-2-yl)propanamide] represents the first amphosteric AURKA inhibitor with a more potent cellular than catalytic inhibition. Furthermore, a kinome screen with 320 wildtype kinases displayed no off-targets at 100 nM for compound 2c [*N*-(5-((4-(1*H*-pyrazole-4-yl)benzyl)amino)-2-fluorophenyl)-3-(furan-2-yl)propanamide] and a good selectivity score of 0.019 at 500 nM, whereas compound 3a [N-(2-fluoro-5-((4-(1-methyl-1*H*-pyrazole-4-yl)benzyl)amino)phenyl)-3-(furan-2-yl)propanamide] showed overall no inhibition. Accordingly, kinase inhibition related off-target effects do not interfere with the observed cellular phenotype, even for compound 2c [*N*-(5-((4-(1*H*-pyrazole-4-yl)benzyl)amino)-2-fluorophenyl)-3-(furan-2-yl)propanamide], since its cellular activity is observed at significantly lower concentrations.

As compound 2c [*N*-(5-((4-(1*H*-pyrazole-4-yl)benzyl)amino)-2-fluorophenyl)-3-(furan-2-yl)propanamide] and compound 3a [*N*-(2-fluoro-5-((4-(1-methyl-1*H*-pyrazole-4-yl)benzyl)amino)phenyl)-3-(furan-2-yl)propanamide] are structurally closely related and based on the observed similarities, the inventors assume that both compounds act via the same mechanism. The inventors were able to detect a strong binding to AURKA for the amphosteric inhibitor compound 2c [*N-*(5-((4-(1*H*-pyrazole-4-yl)benzyl)amino)-2-fluorophenyl)-3-(furan-2-yl)propanamide], in contrast to the PPI modulating compound 3a [N-(2-fluoro-5-((4-(1-methyl-1*H*-pyrazole-4-yl)benzyl)amino)phenyl)-3-(furan-2-yl)propanamide], that showed an improved cellular activity. The highly dynamic character of AURKA could explain the increased cellular efficacy of compound 3a. Interestingly, compound 3a [N-(2-fluoro-5-((4-(1-methyl-1H-pyrazole-4-yl)benzyl)amino)phenyl)-3-(furan-2-yl)propanamide] might occupy an allosteric site crucial for the MYC-AURKA interaction.

**Table 2: Effective concentration (EC) in colony formation assays of the indicated compounds on NMP53^{-/}⁻ and NMCdkn2a^{ARF-/}⁻ cell lines, as well as IC50 values for AURKA kinase inhibition determined by radioisotope filter binding assays (HotSpotSM; ReactionBiology).**

| Compound number | *IUPAC name* | *EC (NMP53* ^{-/-}*) [µM]* | *EC (NMCdkn2a* ^{*ARF-*/}*⁻) [µM]* | IC50 AURKA [nM] |
|---|---|---|---|---|
| 18C | *N*-(5-(((4-(1*H*-Pyrrolo[2,3-b]pyridin-2-yl)pyridin-2-yl)methyl)amino)-2-fluorophenyl)-2-phenylacetamide | 0,25 | 0,5 | > 10000 |
| 80j | *N*-(5-(((4-(1*H*-Pyrrolo[2,3-b]pyridin-2-yl)pyridin-2-yl)methyl)amino)-2-fluorophenyl)-2-cyclohexylacetamide | 0,5 | 1 | - |
| 80k | *N*-(5-(((4-(1*H*-Pyrrolo[2,3-b]pyridin-2-yl)pyridin-2-yl)methyl)amino)-2-fluorophenyl)-2-cyclopropylacetamide | 0,5 | 1 | - |
| 95b | *N*-(5-((4-(1*H*-Pyrazol-4-yl)benzyl)amino)-2-fluorophenyl)-3-phenylpropanamide | 0,5 | 2 | 7,9 |
| 2a | *N*-(5-((4-(1*H*-pyrazole-4-yl)benzyl)amino)-2-fluorophenyl)-3-phenylpropanamide | >4 | >4 | 6899 |
| 97 | *N*-(2-fluoro-5-((4-(1-methyl-1H-pyrazol-4-yl)benzyl)amino)phenyl)-3-phenylpropanamide | 0,25 | 1 | 532,1 |
| 106h | *N*-(5-((4-(1*H*-Pyrazol-4-yl)benzyl)amino)-2-fluorophenyl)-3-(5-methylfuran-2-yl)propanamide | 0,25 | 2 | - |
| 2b | *N*-(5-((4-(1*H*-pyrazole-4-yl)benzyl)amino)-2-fluorophenyl)acetamide | >4 | >4 | - |
| 2c | *N*-(5-((4-(1*H*-pyrazole-4-yl)benzyl)amino)-2-fluorophenyl)-3-(furan-2-yl)propanamide | 0,125 | >1 | 27,6 |
| 106q | *N*-(5-((4-(1*H*-Pyrazol-4-yl)benzyl)amino)-2-fluorophenyl)-3-(1*H*-pyrrol-2-yl)propanamide | 1,0-2,0 | >4 | - |
| 107g | *N*-(2-fluoro-5-((4-(1-methyl-1*H-*pyrazol-4-yl)benzyl)amino)phenyl)-3-(tetrahydrofuran-2-yl)propanamide | 0,125 | 2 | - |
| 107h | *N*-(2-fluoro-5-((4-(1-methyl-1*H-*pyrazol-4-yl)benzyl)amino)phenyl)-3-(5-methylfuran-2-yl)propanamide | 0,125 | 1 | - |
| 3a | *N*-(2-fluoro-5-((4-(1-methyl-1*H-*pyrazole-4-yl)benzyl)amino)phenyl)-3-(furan-2-yl)propanamide | 0,031 | 0,25-0,5 | > 10000 |
| 107m | *N*-(2-fluoro-5-((4-(1-methyl-1*H-*pyrazol-4-yl)benzyl)amino)phenyl)-2-(furan-2-yl)acetamide | 0,125 | 16 | - |
| 107n | *N*-(2-fluoro-5-((4-(1-methyl-1*H-*pyrazol-4-yl)benzyl)amino)phenyl)-3-(furan-3-yl)propanamide | 0,062 | >0,25 | > 10000 |
| 1070 | *N*-(2-fluoro-5-((4-(1-methyl-1*H-*pyrazol-4-yl)benzyl)amino)phenyl)-3-(thiophen-2-yl)propanamide | 0,125 | 1 | > 10000 |
| 107p | *N*-(2-fluoro-5-((4-(1-methyl-1*H-*pyrazol-4-yl)benzyl)amino)phenyl)-3-(thiophen-3-yl)propanamide | 0,062 | >0,25 | > 10000 |
| 107q | *N*-(2-fluoro-5-((4-(1-methyl-1*H-*pyrazol-4-yl)benzyl)amino)phenyl)-3-(1*H*-pyrrol-2-yl)propanamide | 0,5-1 | 4 | - |
| 107r | (*E*)*-N-*(2-fluoro-5-((4-(1-methyl-1*H-*pyrazol-4-yl)benzyl)amino)phenyl)-3-(furan-2-yl)acrylamide | 0,062 | >0,25 | > 10000 |
| 107s | *N*-(2-fluoro-5-((4-(1-methyl-1H-pyrazol-4-yl)benzyl)amino)phenyl)cinnamami de | 0,5 | >1 | >10000 |
| 113a | *N*-(5-((4-(1*H*-Pyrazol-4-yl)benzyl)amino)-2-chlorphenyl)-3-(furan-2-yl)propanamide | 0,5 | >1 | - |
| 124 | *N*-(2-fluoro-5-((4-(1-methyl-1*H-*pyrazol-4-yl)benzyl)oxy)phenyl)-3-phenylpropanamide | 0,25 | >1 | - |
| 4 | *N*-(2-fluoro-5-((4-(1-methyl-1*H-*pyrazole-4-yl)benzyl)amino)phenyl)-3-(furan-2-yl)-N-methylpropanamide | >4 | >4 | - |
| 127b | *N*-(2-fluoro-5-((3-methoxy-4-(1*H-*pyrazol-4-yl)benzyl)amino)phenyl)-3-(furan-2-yl)propanamide | 0,25 | >1 | - |
| 127c | *N*-(2-fluoro-5-((2-fluoro-4-(1*H-*pyrazol-4-yl)benzyl)amino)phenyl)-3-(furan-2-yl)propanamide | 0,125 | 0,5-1 | - |
| 127d | *N*-(2-fluoro-5-((2-methoxy-4-(1*H-*pyrazol-4-yl)benzyl)amino)phenyl)-3-(furan-2-yl)propanamide | 0,25 | 2 | - |
| 127e | *N*-(5-(((6-(1*H*-Pyrazol-4-yl)pyridin-3-yl)methyl)amino)-2-fluorophenyl)-3-(furan-2-yl)-propanamide | 0,5 | >2 | - |
| 128a | *N*-(2-fluoro-5-((3-fluoro-4-(1-methyl-1*H*-pyrazol-4-yl)benzyl)amino)phenyl)-3-(furan-2-yl)propanamide | 0,032 | 0,125-0,25 | - |
| 128b | *N*-(2-fluoro-5-((3-methoxy-4-(1-methyl-1*H*-pyrazol-4-yl)benzyl)amino)phenyl)-3-(furan-2-yl)propanamide | 0,062 | >0,5 | - |
| 128c | *N*-(2-fluoro-5-((2-fluoro-4-(1-methyl-1*H*-pyrazol-4-yl)benzyl)amino)phenyl)-3-(furan-2-yl)propanamide | 0,125 | 0,5-1 | - |
| 128d | *N*-(2-fluoro-5-((2-methoxy-4-(1-methyl-1*H*-pyrazol-4-yl)benzyl)amino)phenyl)-3-(furan-2-yl)propanamide | 0,031 | >0,5 | - |
| 128e | *N*-(2-fluoro-5-(((6-(1-methyl-1*H-*pyrazol-4-yl)pyridin-3-yl)methyl)amino)phenyl)-3-(furan-2-yl)propanamide | 0,25 | >2 | - |
| 130 | *N*-(2-fluoro-5-(((5-(1-methyl-1*H-*pyrazol-4-yl)pyridin-2-yl)methyl)amino)phenyl)-3-phenylpropanamide | 0,5 | >1 | - |
| 139 | *N*-(5-((4-(3,5-Dimethylisoxazol-4-yl)benzyl)amino)-2-fluorophenyl)-3-(furan-2-yl)propanamide | 0,5 | 1 | - |
| 164 | *N*-(5-((4-(1-Acetyl-1*H*-pyrazol-4-yl)benzyl)amino)-2-fluorophenyl)-3-(furan-2-yl)propanamide | 0,125 | >1 | - |
| 167 | *N*-(5-((4-(1-(2-Aminoethyl)-1*H-*pyrazol-4-yl)benzyl)amino)-2-fluorophenyl)-3-(furan-2-yl)propanamide | 0,5 | 1 | - |
| 144 | *N*-(5-((4-(3-Amino-1*H*-pyrazol-4-yl)benzyl)amino)-2-fluorophenyl)-3-(furan-2-yl)propanamide | 0,25 | >1 | - |
| 145 | *N*-(5-((4-(3-Amino-1-methyl-1*H-*pyrazol-4-yl)benzyl)amino)-2-fluorophenyl)-3-(furan-2-yl)propanamide | 0,25 | >1 | - |
| 146 | *N*-(5-((4-(5-Amino-1-methyl-1*H-*pyrazol-4-yl)benzyl)amino)-2-fluorophenyl)-3-(furan-2-yl)propanamide | 0,125 | >1 | - |
| 155 | *N*-(2-fluoro-5-((4-((1-methyl-1*H-*pyrazol-3-yl)amino)benzyl)amino)phenyl)-3-(furan-2-yl)propanamide | 0,25 | >1 | - |
| 3b | *N*-(5-((4-(1-ethyl-1*H*-pyrazole-4-yl)benzyl)amino)-2-fluorophenyl)-3-(furan-2-yl)propanamide | 0,125 | 0,25 | - |
| 3c | *N*-(2-fluoro-5-((4-(1-propyl-1*H-*pyrazole-4-yl)benzyl)amino)phenyl)-3-(furan-2-yl)propanamide | 4 | >4 | - |
| 3d | *N*-(2-fluoro-5-((4-(1-isopropyl-1*H-*pyrazole-4-yl)benzyl)amino)phenyl)-3-(furan-2-yl)propanamide | 4 | 4 | - |

Table 2 summarizes the EC values of different compounds of this invention determined by colony formation assays on NMP53^{-/-} and NMCdkn2a^{ARF-/-} cells, as well the corresponding IC₅₀ values for the kinase inhibition of AURKA, determined by radioisotope filter binding assay (HotSpotSM; ReactionBiology). Most compounds further show a strong selectivity for NMP53^{-/-}cells, which is indicated by a lower EC concentration of a particular compound required for inhibiting cell growth of NMP53^{-/-} cells compared to NMCdkn2a^{ARF-/-} cells. A higher IC₅₀ value indicates that the kinase activity of AURKA is only affected by the compound at higher concentrations as those required for the cellular effects.

### Example 5: The compounds of this invention induce an increased interaction of AURKA with TPX2, and the formation of abnormal spindles

Tumor cells (NMP53^{-/-} cells) expressing a doxycycline-inducible FLAG-AURKA construct were kept on doxycycline for 24 hours, synchronized with 50 ng/ml nocodazole for 6 hours and treated with two of the developed compounds of this invention (3a and 2c), or DMSO as a control. Then, co-immunoprecipitations (Co-IP) were carried out with an antibody against FLAG. A DMSO-treated sample without FLAG-AURKA induction served as a control. The AURKA and TPX2 levels of the Co-IP samples and their input were determined by western blotting. The compounds of this invention largely enhance the interaction of AURKA with TPX2, as can be seen in the strongly enhanced TPX2 signal in western blot analysis in the presence of the compounds (Figure 7).

The AURKA compounds of this invention trigger the formation of abnormal (e.g. multipolar and/or monopolar) spindles in tumor cell lines (NMP53^{-/-} cells) (Figure 8). The amount of normal and abnormal spindles of a tumor cell line (NMP53^{-/-} cells) after different treatment times with the compound 3a or DMSO as a control were quantified in immunofluorescence stainings and indicate that the AURKA targeting compounds of this invention induce the formation of abnormal spindles within a few minutes.

The references are:
1. Li, J.-S. et al. Solvent-, and Catalyst-free Acylation of Anilines with Meldrum's Acids: ANeat Access to Anilides. ChemistrySelect 2, 1770-1773, doi:10.1002/slct.201601965 (2017).
2. Dauch, D. et al. A MYC-aurora kinase A protein complex represents an actionable drug target in p53-altered liver cancer. Nat Med 22, 744-753, doi:10.1038/nm.4107 (2016).
3. Mohane, S. C. et al. Fast-Forwarding Hit to Lead: Aurora and Epidermal Growth Factor Receptor Kinase Inhibitor Lead Identification. J. Med. Chem. 53, 4980-4988, doi:10.1021/jm1000198 (2010).
4. Cheung, C. H. A. et al. BPR1K653, a Novel Aurora Kinase Inhibitor, Exhibits Potent AntiProliferative Activity in MDR1 (P-gp170)-Mediated Multidrug-Resistant Cancer Cells. PLoS ONE, 6(8), e23485. doi:10.1371/journal.pone.0023485 (2011).
5. Yung, C. H. et al. Discovery of BPR1K871, a quinazoline based, multi-kinase inhibitor for the treatment of AML and solid tumors: Rational design, synthesis, in vitro and in vivo evaluation. ONCOTARGET 7, no. 52, 86239-86256, doi:10.18632/oncotarget.13369 (2016).

## Claims

1. A compound wherein the compound comprises an anilide moiety according to formula (III): 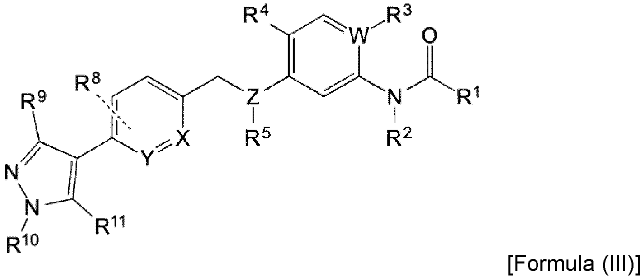 wherein
W is C, or N, if W = N, R³ is dispensed with;
X and Y are independently selected from C and N;
Z is N, or O; if Z = O, R⁵ is dispensed with;
R¹ is selected from -CH₂-aryl; -CH₂-(hetero)aryl; -CH₂-CH₂-aryl; -CH₂-CH₂-(hetero)aryl; -N(R²)-CH2-aryl; and -N(R²)-CH2-heteroaryl
R² is H, or Me;
R³ and R⁴ are independently selected from H, F, Cl and Me;
R⁵ is H;
R⁸ is one or more substituents selected from H, F and methoxy, preferably two F or two methoxy substituents;
R⁹ is H, or alkyl;
R¹⁰ is selected from alkyl, aminoalkyl, morpholinalkyl, acetyl, tosyl, and benzyl;
R¹¹ is H, NH₂, or aminobenzyl;
and the pharmaceutically acceptable salts, solvates and optical isomers thereof.

2. The compound according to claim 1, wherein R¹ is selected from phenethyl, and (furan-2-yl)ethyl, preferably wherein R¹ is (furan-2-yl)ethyl.

3. The compound according to claim 1 or 2, wherein R² and R⁴ is H, and R³ is F, preferably wherein Z is N and R⁵ is H.

4. The compound according to any one of claims 1 to 3, wherein R⁹ and R¹¹ is H, preferably wherein R¹⁰ is H or methyl, more preferably wherein the compound is [*N*-(5-((4-(1*H*-pyrazole-4-yl)benzyl)amino)-2-fluorophenyl)-3-(furan-2-yl)propanamide], [N-(2-fluoro-5-((4-(1-methyl-1H-pyrazole-4-yl)benzyl)amino)phenyl)-3-(furan-2-yl)propanamide], or the pharmaceutically acceptable salts, solvates and optical isomers thereof.

5. The compound according to any one of claims 1 to 4, wherein the compound is capable of binding, preferably of specifically binding, to a serine/threonine kinase, more preferably wherein the serine/threonine kinase is Aurora Kinase A (AURKA), or a variant thereof, and wherein the compound modulates, stabilizes and/or destabilizes, preferably specifically modulates, stabilizes and/or destabilizes, the conformation of the serine/threonine kinase, preferably of AURKA, or the variant thereof.

6. The compound according to any one of claims 1 to 5, wherein the compound modulates, mimics, stabilizes and/or destabilizes, preferably specifically modulates, mimics, stabilizes and/or destabilizes, an interaction of the serine/threonine kinase, preferably of AURKA, or the variant thereof, with at least one binding protein, more preferably wherein the serine/threonine kinase is AURKA, or a variant thereof, and wherein the at least one binding protein is TPX2 and/or MYC, even more preferably wherein the compound stabilizes and/or increases, most preferably specifically stabilizes and/or increases, the interaction of AURKA, or the variant thereof, with TPX2, and/or wherein the compound destabilizes and/or decreases, most preferably specifically destabilizes and/or decreases, the interaction of AURKA, or the variant thereof, with MYC.

7. The compound according to any one of claims 1 to 6, wherein the compound destabilizes and/or decreases, preferably specifically destabilizes and/or decreases, the level and/or the activity of MYC in a cell, and/or wherein the compound induces and/or enhances an abnormal and/or defect mitosis of a cell, and wherein the compound induces and/or enhances the formation of at least one abnormal and/or defect spindle in the cell.

8. A pharmaceutical composition comprising a compound according to any one of claims 1 to 7, and a pharmaceutically acceptable carrier, stabilizer and/or excipient.

9. A compound according to any one of claims 1 to 7, or a pharmaceutical composition according to claim 8, for use in medicine.

10. A compound according to any one of claims 1 to 7, or a pharmaceutical composition according to claim 8, for use in the prevention and/or treatment of a proliferative disease, such as cancer, preferably wherein the cancer is a solid cancer, such as a cancer stemming from any solid organ tissue, or a liquid cancer.

11. The compound or the pharmaceutical composition for use according to claim 10, wherein the cancer is liver cancer, hepatocellular carcinoma (HCC), lung cancer, small cell lung cancer (SCLC), bladder cancer, ovarian cancer, uterine cancer, endometrial cancer, breast cancer, gastric cancer, urinary bladder cancer, renal cancer, pancreatic cancer, stomach cancer, cervical cancer, cephalic cancer, thyroid cancer, esophageal cancer, medulloblastoma, head and neck cancer, lymphoma, leukemia, acute myeloid leukemia, acute lymphocytic leukemia, chronic lymphocytic leukemia, chronic myeloid cancer, prostate cancer, salivary gland cancer, bone cancer, brain cancer, glioma, colon cancer, rectal cancer, colorectal cancer, kidney cancer, skin cancer, melanoma, glioblastoma, squamous cell carcinoma, pleomorphic adenoma, endometrioma, nasopharynx cancer, small intestine cancer, testicular cancer, Hodgkin lymphoma, non-Hodgkin lymphoma, cancer of the anus, cancer of the anal canal, cancer of the anorectum, cancer of the oropharynx, vaginal carcinoma, vulval carcinoma, enteric cancer, endocrine gland cancer, adrenal cancer, urethral cancer, lymphocytoma, cystic cancer, nephritic cancer, hydrouretic cancer, renal cell carcinoma, renal pelvic carcinoma, hypophyseal adenomatosis, adenocarcinoma, and/or a MYC-dependent tumor, preferably wherein the cancer is liver cancer, in particular hepatocellular carcinoma (HCC), or lung cancer, in particular small cell lung cancer (SCLC).

12. The compound or the pharmaceutical composition for use according to claim 10 or 11, wherein the compound is modulating, mimicking, stabilizing and/or destabilizing, preferably specifically modulating, mimicking, stabilizing and/or destabilizing, an interaction of a serine/threonine kinase, preferably AURKA, or a variant thereof, with at least one binding protein in a cell, such as in a human cancer cell, preferably wherein the serine/threonine kinase is AURKA, or a variant thereof, and the at least one binding protein is MYC, and/or TPX2, thereby preventing and/or treating the proliferative disease, such as cancer, in a subject, optionally wherein the modulating, mimicking, stabilizing and/or destabilizing, preferably the specifically modulating, mimicking, stabilizing and/or destabilizing, of the interaction of the serine/threonine kinase, preferably of AURKA, or the variant thereof, with the at least one binding protein, induces the death of at least one tumor cell in the subject.

13. In vitro use of a compound according to any one of claims 1 to 7 or 9 to 12, or a pharmaceutical composition according any one of claims 8 to 12, in selectively modulating the interaction of a serine/threonine kinase, or a variant thereof, with at least one binding protein, preferably wherein the serine/threonine kinase is AURKA, or the variant thereof, and the at least one binding protein is Myc, and/or TPX2, and/or in modulating the interactome of the serine/threonine kinase, or the variant thereof, such as AURKA.

## Patentansprüche

1. Verbindung, wobei die Verbindung eine Anilideinheit gemäß Formel (III) umfasst: 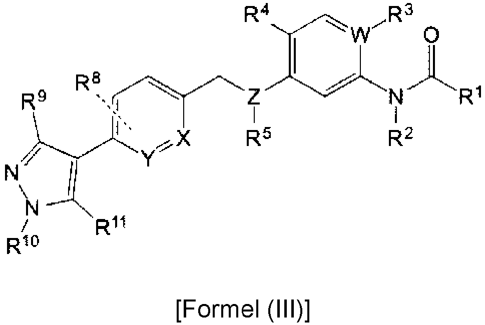 wobei
W C oder N ist, wenn W = N, auf R³ verzichtet wird;
X und Y unabhängig voneinander ausgewählt sind aus C und N;
Z N oder O ist; wenn Z = O, auf R⁵ verzichtet wird;
R¹ ausgewählt ist aus -CH₂-Aryl; -CH₂-(Hetero)aryl; -CH₂-CH₂-Aryl; -CH₂-CH₂-(Hetero)aryl; -N(R²)-CH2-Aryl; und -N(R²)-CH2-Heteroaryl
R² H oder Me ist;
R³ und R⁴ unabhängig voneinander ausgewählt sind aus H, F, Cl und Me;
R⁵ H ist;
R⁸ ein oder mehrere Substituenten, ausgewählt aus H, F und Methoxy, vorzugsweise zwei F- oder zwei Methoxy-Substituenten ist;
R⁹ H oder Alkyl ist;
R¹⁰ ausgewählt ist aus Alkyl, Aminoalkyl, Morpholinalkyl, Acetyl, Tosyl und Benzyl;
R¹¹ H, NH₂ oder Aminobenzyl ist;
und die pharmazeutisch verträglichen Salze, Solvate und optischen Isomere davon.

2. Verbindung nach Anspruch 1, wobei R¹ ausgewählt ist aus Phenethyl und (Furan-2-yl)ethyl, vorzugsweise wobei R¹ (Furan-2-yl)ethyl ist.

3. Verbindung nach Anspruch 1 oder 2, wobei R² und R⁴ H ist und R³ F ist, vorzugsweise wobei Z N ist und R⁵ H ist.

4. Verbindung nach einem der Ansprüche 1 bis 3, wobei R⁹ und R¹¹ H ist, vorzugsweise wobei R¹⁰ H oder Methyl ist, mehr bevorzugt wobei die Verbindung [*N*-(5-((4-(1/*H*-pyrazol-4-yl)benzyl)amino)-2-fluorphenyl)-3-(furan-2-yl)propanamid], [N-(2-Fluor-5-((4-(1-methyl-1/H-pyrazol-4-yl)benzyl)amino)phenyl)-3-(furan-2-yl)propanamid] ist oder die pharmazeutisch verträglichen Salze, Solvate und optischen Isomere davon.

5. Verbindung nach einem der Ansprüche 1 bis 4, wobei die Verbindung in der Lage ist, an eine Serin/Threonin-Kinase zu binden, vorzugsweise spezifisch zu binden, mehr bevorzugt wobei die Serin/Threonin-Kinase Aurora-Kinase A (AURKA) oder eine Variante davon ist, und wobei die Verbindung die Konformation der Serin/Threonin-Kinase, vorzugsweise von AURKA, oder der Variante davon, moduliert, stabilisiert und/oder destabilisiert, vorzugsweise spezifisch moduliert, stabilisiert und/oder destabilisiert.

6. Verbindung nach einem der Ansprüche 1 bis 5, wobei die Verbindung eine Interaktion der Serin/Threonin-Kinase, vorzugsweise von AURKA oder der Variante davon, mit mindestens einem Bindungsprotein moduliert, nachahmt, stabilisiert und/oder destabilisiert, vorzugsweise spezifisch moduliert, nachahmt, stabilisiert und/oder destabilisiert, mehr bevorzugt wobei die Serin/Threonin-Kinase AURKA oder eine Variante davon ist, und wobei das mindestens eine Bindungsprotein TPX2 und/oder MYC ist, noch mehr bevorzugt wobei die Verbindung die Interaktion von AURKA oder der Variante davon mit TPX2 stabilisiert und/oder erhöht, am meisten bevorzugt spezifisch stabilisiert und/oder erhöht, und/oder wobei die Verbindung die Interaktion von AURKA oder der Variante davon mit MYC destabilisiert und/oder verringert, am meisten bevorzugt spezifisch destabilisiert und/oder verringert.

7. Verbindung nach einem der Ansprüche 1 bis 6, wobei die Verbindung den Spiegel und/oder die Aktivität von MYC in einer Zelle destabilisiert und/oder verringert, vorzugsweise spezifisch destabilisiert und/oder verringert, und/oder wobei die Verbindung eine anormale und/oder defekte Mitose einer Zelle induziert und/oder verstärkt, und wobei die Verbindung die Bildung mindestens einer anormalen und/oder defekten Spindel in der Zelle induziert und/oder verstärkt.

8. Pharmazeutische Zusammensetzung, umfassend eine Verbindung nach einem der Ansprüche 1 bis 7, und einen pharmazeutisch verträglichen Träger, Stabilisator und/oder Exzipienten.

9. Verbindung nach einem der Ansprüche 1 bis 7 oder pharmazeutische Zusammensetzung nach Anspruch 8 zur Verwendung in der Medizin.

10. Verbindung nach einem der Ansprüche 1 bis 7 oder pharmazeutische Zusammensetzung nach Anspruch 8 zur Verwendung bei der Vorbeugung und/oder Behandlung einer proliferativen Erkrankung wie Krebs, vorzugsweise wobei der Krebs ein solider Krebs, wie ein Krebs, der von einem festen Organgewebe ausgeht, oder ein flüssiger Krebs ist.

11. Verbindung oder pharmazeutische Zusammensetzung zur Verwendung nach Anspruch 10, wobei der Krebs Leberkrebs, hepatozelluläres Karzinom (HCC), Lungenkrebs, kleinzelliger Lungenkrebs (SCLC), Blasenkrebs, Eierstockkrebs, Gebärmutterkrebs, Endometriumkarzinom, Brustkrebs, Magenkrebs, Harnblasenkrebs, Nierenkrebs, Bauchspeicheldrüsenkrebs, Magenkrebs, Gebärmutterhalskrebs, Kopfkrebs, Schilddrüsenkrebs, Speiseröhrenkrebs, Medulloblastom, Kopf-Hals-Krebs, Lymphom, Leukämie, akute myeloische Leukämie, akute lymphatische Leukämie, chronische lymphatische Leukämie, chronischer myeloischer Krebs, Prostatakrebs, Speicheldrüsenkrebs, Knochenkrebs, Hirntumor, Gliom, Dickdarmkrebs, Mastdarmkrebs, kolorektaler Krebs, Nierenkrebs, Hautkrebs, Melanom, Glioblastom, Plattenepithelkarzinom, pleomorphes Adenom, Endometriom, Nasopharynxkrebs, Dünndarmkrebs, Hodenkrebs, Hodgkin-Lymphom, Non-Hodgkin-Lymphom, Anuskrebs, Analkanalkrebs, Anorektalkrebs, Oropharynxkrebs, Vaginalkarzinom, Vulvakarzinom, Darmkrebs, endokriner Drüsenkrebs, Nebennierenkrebs, Harnröhrenkrebs, Lymphozytom, Zysten krebs, Nierenkrebs, Hydrouresekrebs, Nierenzellkarzinom, Nierenbeckenkarzinom, Hypophysenadenomatose, Adenokarzinom und/oder ein MYC-abhängiger Tumor ist, vorzugsweise wobei der Krebs Leberkrebs, insbesondere hepatozelluläres Karzinom (HCC), oder Lungenkrebs, insbesondere kleinzelliges Lungenkarzinom (SCLC), ist.

12. Verbindung oder pharmazeutische Zusammensetzung zur Verwendung nach Anspruch 10 oder 11, wobei die Verbindung eine Interaktion einer Serin/Threonin-Kinase, vorzugsweise AURKA, oder einer Variante davon, mit mindestens einem Bindungsprotein in einer Zelle, wie in einer menschlichen Krebszelle, moduliert, nachahmt, stabilisiert und/oder destabilisiert, vorzugsweise spezifisch moduliert, nachahmt, stabilisiert und/oder destabilisiert, vorzugsweise wobei die Serin/Threonin-Kinase AURKA oder eine Variante davon ist und das mindestens eine Bindungsprotein MYC und/oder TPX2 ist, wodurch die proliferative Erkrankung, wie Krebs, bei einem Subjekt verhindert und/oder behandelt wird, wobei wahlweise das Modulieren, Nachahmen, Stabilisieren und/oder Destabilisieren, vorzugsweise das spezifische Modulieren, Nachahmen, Stabilisieren und/oder Destabilisieren der Interaktion der Serin/Threonin-Kinase, vorzugsweise von AURKA, oder der Variante davon, mit dem mindestens einen Bindungsprotein den Tod von mindestens einer Tumorzelle in dem Subjekt auslöst.

13. In-vitro-Verwendung einer Verbindung nach einem der Ansprüche 1 bis 7 oder 9 bis 12 oder einer pharmazeutischen Zusammensetzung nach einem der Ansprüche 8 bis 12 zum selektiven Modulieren der Interaktion einer Serin/Threonin-Kinase oder einer Variante davon mit mindestens einem Bindungsprotein, vorzugsweise wobei die Serin/Threonin-Kinase AURKA oder die Variante davon ist und das mindestens eine Bindungsprotein Myc und/oder TPX2 ist, und/oder zum Modulieren des Interaktoms der Serin/Threonin-Kinase oder der Variante davon, wie AURKA.

## Revendications

1. Composé dans lequel le composé comprend une partie anilide selon la formule (III) : 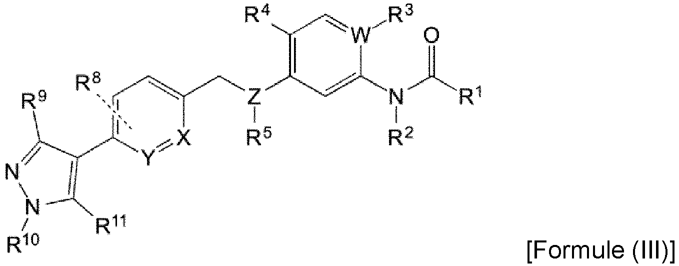 dans lequel
W est C, ou N, si W = N, R³ est supprimé ;
X et Y sont choisis indépendamment parmi C et N ;
Z est N ou O ; si Z = O, R⁵ est supprimé ;
R¹ est choisi parmi -CH₂-aryle ; -CH₂-(hétéro)aryle ; -CH₂-CH₂-aryle ; - CH₂-CH₂-(hétéro)aryle ; -N(R²)-CH2-aryle ; et -N(R²)-CH2-hétéroaryle
R² est H, ou Me ;
R³ et R⁴ sont indépendamment choisis parmi H, F, Cl et Me ;
R⁵ est H ;
R⁸ est un ou plusieurs substituants choisis parmi H, F et méthoxy, de préférence, deux substituants F ou deux substituants méthoxy ;
R⁹ est H ou un alkyle ;
R¹⁰ est choisi parmi les alkyles, les aminoalcoyles, les morpholinalkyles, les acétyles, les tosyles et les benzyles ;
R¹¹ est H, NH₂ ou aminobenzyle ;
et des sels, solvates et isomères optiques pharmaceutiquement acceptables de ceux-ci.

2. Composé selon la revendication 1, dans lequel R¹ est choisi parmi le phénéthyle et le (furan-2-yl)éthyle, de préférence, dans lequel R¹ est le (furan-2-yl)éthyle.

3. Composé selon la revendication 1 ou 2, dans lequel R² et R⁴ sont H, et R³ est F, de préférence, dans lequel Z est N et R⁵ est H.

4. Composé selon l'une quelconque des revendications 1 à 3, dans lequel R⁹ et R¹¹ est H, de préférence, dans lequel R¹⁰ est H ou méthyle, plus préférablement, dans lequel le composé est [A/-(5-((4-(1/7-pyrazole- 4-yl)benzyl)amino)-2-fluorophényl)-3-(furan-2-yl)propanamide], [A/-(2-fluoro-5-((4-(1-méthyl-1/7-pyrazole-4-yl)benzyl)amino)phényl)-3-(furan-2-yl)propanamide], ou des sels, solvates et isomères optiques pharmaceutiquement acceptables de ceux-ci.

5. Composé selon l'une quelconque des revendications 1 à 4, dans lequel le composé est capable de se lier, de préférence, de se lier spécifiquement, à une sérine/thréonine kinase, de préférence, dans lequel la sérine/thréonine kinase est l'Aurora Kinase A (AURKA), ou une variante de celle-ci, et dans lequel le composé module, stabilise et/ou déstabilise, de préférence module spécifiquement, stabilise et/ou déstabilise, la conformation de la sérine/thréonine kinase, de préférence, de l'AURKA, ou de la variante de celle-ci.

6. Composé selon l'une quelconque des revendications 1 à 5, dans lequel le composé module, imite, stabilise et/ou déstabilise, de préférence module, imite, stabilise et/ou déstabilise spécifiquement, une interaction de la sérine/thréonine kinase, de préférence, de l'AURKA, ou de la variante de celle-ci, avec au moins une protéine de liaison, plus préférentiellement dans lequel la sérine/thréonine kinase est l'AURKA, ou de la variante de celle-ci, et dans lequel l'au moins une protéine de liaison est TPX2 et/ou le Myc, plus préférablement, dans lequel le composé stabilise et/ou augmente, de préférence, stabilise spécifiquement et/ou augmente, l'interaction d'AURKA, ou de la variante de celle-ci, avec TPX2, et/ou dans lequel le composé déstabilise et/ou diminue, de préférence, déstabilise spécifiquement et/ou diminue, l'interaction de l'AURKA, ou de la variante de celle-ci, avec le Myc.

7. Composé selon l'une quelconque des revendications 1 à 6, dans lequel le composé déstabilise et/ou diminue, de préférence, déstabilise et/ou diminue spécifiquement, le niveau et/ou l'activité du Myc dans une cellule, et/ou dans lequel le composé induit et/ou renforce une mitose anormale et/ou défectueuse d'une cellule, et dans lequel le composé induit et/ou renforce la formation d'au moins un fuseau anormal et/ou défectueux dans la cellule.

8. Composition pharmaceutique comprenant un composé selon l'une quelconque des revendications 1 à 7, et un support, un stabilisateur et/ou un excipient pharmaceutiquement acceptable.

9. Composé selon l'une quelconque des revendications 1 à 7, ou composition pharmaceutique selon la revendication 8, pour utilisation en médecine.

10. Composé selon l'une quelconque des revendications 1 à 7, ou composition pharmaceutique selon la revendication 8, pour utilisation dans la prévention et/ou le traitement d'une maladie proliférative, telle que le cancer, de préférence, lorsque le cancer est un cancer solide, tel qu'un cancer provenant d'un tissu organique solide, ou un cancer liquide.

11. Composé ou composition pharmaceutique pour utilisation selon la revendication 10, dans lequel le cancer est le cancer du foie, le carcinome hépatocellulaire (CHC), le cancer du poumon, le cancer bronchique à petites cellules (SCLC), le cancer de la vessie, le cancer de l'ovaire, le cancer de l'utérus, le cancer de l'endomètre, le cancer du sein, le cancer gastrique, le cancer de la vessie, le cancer du rein, le cancer du pancréas, le cancer de l'estomac, le cancer du col de l'utérus, le cancer du cerveau, le cancer de la thyroïde, le cancer de l'œsophage, le médulloblastome, le cancer de la tête et du cou, le lymphome, la leucémie, la leucémie aiguë myéloïde, la leucémie aiguë lymphoblastique, la leucémie lymphoïde chronique, le cancer myéloïde chronique, le cancer de la prostate, le cancer des glandes salivaires, le cancer des os, le cancer du cerveau, le gliome, le cancer du côlon, le cancer du rectum, le cancer colorectal, le cancer du rein, le cancer de la peau, le mélanome, le glioblastome, le carcinome épidermoïde, l'adénome pléomorphe, l'endométriome, le cancer du nasopharynx, le cancer de l'intestin grêle, le cancer du testicule, le lymphome hodgkinien, le lymphome non hodgkinien, le cancer de l'anus, le cancer du canal anal, le cancer de l'anorectum, le cancer de l'oropharynx, le carcinome vaginal, le carcinome vulvaire, le cancer entérique, le cancer des glandes endocrines, le cancer des glandes surrénales, le cancer de l'urètre, le cancer de l'intestin grêle, le cancer de l'intestin grêle, le cancer de l'intestin grêle, le cancer de l'intestin grêle, le cancer de l'intestin grêle, etc, le cancer des glandes endocrines, le cancer des glandes surrénales, le cancer de l'urètre, le lymphocytome, le cancer kystique, le cancer néphritique, le cancer hydronéphrose, le carcinome des cellules rénales, le carcinome pelvien rénal, l'adénomatose hypophysaire, l'adénocarcinome, et/ou la tumeur dépendante du Myc, de préférence dans lequel le cancer est un cancer du foie, en particulier, un carcinome hépatocellulaire (CHC), ou un cancer du poumon, en particulier, un cancer du poumon à petites cellules (SCLC).

12. Composé ou composition pharmaceutique pour utilisation selon la revendication 10 ou 11, dans lequel le composé module, imite, stabilise et/ou déstabilise, de préférence module, imite, stabilise et/ou déstabilise spécifiquement, une interaction d'une sérine/thréonine kinase, de préférence, de l'AURKA, ou de la variante de celle-ci, avec au moins une protéine de liaison dans une cellule, telle qu'une cellule cancéreuse humaine, de préférence, dans lequel la sérine/thréonine kinase est l'AURKA, ou de la variante de celle-ci, et l'au moins une protéine de liaison est le Myc, et/ou TPX2, prévenant et/ou traitant ainsi la maladie proliférative, telle que le cancer, chez un sujet, éventuellement, dans lequel la modulation, le mimétisme, la stabilisation et/ou la déstabilisation, de préférence la modulation, le mimétisme, la stabilisation et/ou la déstabilisation spécifiques de l'interaction de la sérine/thréonine kinase, de préférence, de l'AURKA, ou de la variante de celle-ci, avec l'au moins une protéine de liaison, induit la mort d'au moins une cellule tumorale chez le sujet.

13. Utilisation in vitro d'un composé selon l'une quelconque des revendications 1 à 7 ou 9 à 12, ou composition pharmaceutique selon l'une quelconque des revendications 8 à 12, pour moduler sélectivement l'interaction d'une sérine/thréonine kinase, ou d'une variante de celle-ci, avec au moins une protéine de liaison, de préférence, lorsque la sérine/thréonine kinase est l'AURKA, ou de la variante de celle-ci, et que l'au moins une protéine de liaison est le Myc, et/ou TPX2, et/ou pour moduler l'interactome de la sérine/thréonine kinase, ou de la variante de celui-ci, telle que l'AURKA.
